# FASCICULE DE BREVET EUROPEEN

(11) **EP 4 577 112 B1**
(45) Date de publication et mention de la délivrance du brevet: **26.08.2026**
(21) Numéro de dépôt: 23762397.0
(22) Date de dépôt: 27.08.2023
(51) Int. Cl.: A61B 5/145, G01N 33/00, G01N 33/49, G01N 33/497, A61B 5/083, A61B 5/1455, A61B 5/1477, A61B 5/00

(54) **PROCÉDÉ D'ESTIMATION D'UNE CONCENTRATION DE GAZ DÉGAGÉ PAR UN MILIEU**
VERFAHREN ZUR SCHÄTZUNG EINER KONZENTRATION EINES VON EINEM MEDIUM EMITTIERTEN GASES
METHOD FOR ESTIMATING A CONCENTRATION OF GAS EMITTED BY A MEDIUM

(30) Priorité: 28.08.2022 FR 2208595; 03.12.2022 FR 2212730
(43) Date de publication de la demande: 02.07.2025
(73) Titulaire: Commissariat à l'Energie Atomique et aux Energies Alternatives, 75015 Paris (FR); Institut Polytechnique de Grenoble, 38000 Grenoble (FR); Université Grenoble Alpes, 38400 Saint-Martin-d'Hères (FR); Centre National de la Recherche Scientifique, 75016 Paris 16 (FR)
(72) Inventeur: GRANGEAT, Pierre, 38054 Grenoble cedex 09 (FR); COMSA, Maria-Paula, 38054 Grenoble Cedex 09 (FR); KOENIG, Anne, 38054 Grenoble Cedex (FR); PHLYPO, Ronald, 38402 Saint Martin D'Hères (FR)
(74) Mandataire: INNOV-GROUP
(86) Numéro de dépôt international: PCT/EP2023/073458
(87) Numéro de publication internationale: WO 2024/046930

(56) Documents cités:
- WO-A1-2017/023500
- WO-A1-2020/249466
- JP-A- 2010 148 692
- "Abstracts", FUEL AND ENERGY ABSTRACTS, ELSEVIER, AMSTERDAM, NL, vol. 61, no. 6, 14 October 2020 (2020-10-14), pages 562 - 661, XP086295297, ISSN: 0140-6701, [retrieved on 20201014], DOI: 10.1016/J.FUELENEAB.2020.09.002

## Description

### DOMAINE TECHNIQUE

Le domaine technique de l'invention est la mesure d'un gaz dégagé par un milieu à l'aide d'un dispositif compact. Le dispositif compact est appliqué contre le milieu. Le milieu peut être la peau d'un être vivant. Le procédé permet alors d'estimer une teneur d'un gaz dégagé par le milieu, Le gaz peut notamment être du dioxyde de carbone, pour des applications de capnométrie. Il s'agit alors d'estimer la teneur de dioxyde de carbone dissous dans le sang. Le milieu peut être un milieu liquide, par exemple de l'eau, ou du lisier. Le gaz peut être du dioxyde de carbone ou du méthane. Le milieu peut également être un milieu végétal, auquel cas le dispositif peut être utilisé pour étudier la respiration du milieu.

### ART ANTERIEUR

Certaines maladies respiratoires affectent les échanges gazeux entre le sang et l'air expiré. Le sang contient des gaz dissous, entre autres l'oxygène et le dioxyde de carbone (CO₂), dont les pressions partielles respectives reflètent les échanges gazeux se produisant au niveau des poumons et des organes.

Pour évaluer la concentration de CO₂ dissous dans le sang, on peut avoir recours à un prélèvement sanguin. Il s'agit alors d'une méthode invasive, pouvant être douloureuse et délicate à appliquer, en particulier en néonatologie. De plus, elle ne peut être appliquée que de façon ponctuelle. En dépit de ces inconvénients, sa fiabilité est validée par le corps médical et elle constitue une méthode de référence. Une autre méthode consiste à estimer la teneur du sang en CO₂ de façon non invasive, en effectuant une mesure de la pression partielle de CO₂ diffusant à travers les tissus, et notamment la peau. Cette méthode est désignée par le terme de capnométrie transcutanée.

Dans le sang, le gaz carbonique est dissous suivant une espèce moléculaire (CO₂), et suivant deux espèces ioniques : les ions carbonates CO₃²⁻ et bicarbonates HCO₃⁻. L'équilibre entre ces espèces dépend du pH du sang, car les ions carbonates et bicarbonates sont en équilibre avec les ions hydrogène H⁺. Ainsi, la concentration des ions carbonates ou bicarbonates influence le pH du sang. Une augmentation de la concentration de CO₂ dissous (hypercapnie), se traduit par une augmentation de la quantité d'ions carbonates et bicarbonates, et, par équilibre, des ions hydrogène, ce qui engendre une diminution du pH du sang, ou acidose. Une augmentation de la concentration de CO₂ peut survenir lorsque l'élimination du CO₂ par les voies respiratoires est insuffisante, par exemple dans le cas de maladies pulmonaires obstructives chroniques, (COPD) ou de maladies infectieuses affectant les poumons, un exemple étant une infection due au COVID 19.

Inversement, une diminution de la concentration de CO₂ dissous (hypocapnie) abaisse la concentration d'ions hydrogène, ce qui entraîne une augmentation du pH, ou alcalose. Une hypocapnie peut être causée par exemple par une hyperventilation associée à une augmentation de la fréquence respiratoire. La survenue d'une acidose ou d'une alcalose peut avoir des conséquences sur le métabolisme. Ainsi, la concentration de CO₂ dans le sang est un paramètres vital important, qu'il convient de surveiller régulièrement pour certains patients à risque.

Le suivi de la concentration de CO₂ peut également concerner des patients en réanimation, ou chez des nouveaux-nés placés en incubateur. Cela peut également trouver des applications dans le suivi d'efforts, l'activité physique favorisant la production de gaz carbonique.

Ce type d'analyse basé sur des mesures transcutanées a été introduit dans les années 1980. Les mesures transcutanées permet d'effectuer un suivi continu, par exemple pour suivre les effets immédiats d'une prise en charge thérapeutique influençant la concentration du CO₂.dans le sang. Elle peut également permettre de déterminer les instants auxquels une quantification plus précise, par prélèvement sanguin, est préférable. On comprend ainsi que les méthodes invasives et non invasives peuvent être complémentaires : l'une est précise est ponctuelle, tandis que l'autre peut être mise en œuvre en œuvre de façon continue pour un suivi longitudinal.

Le document WO2017/023500 décrit un dispositif permettant une mesure d'un gaz, en l'occurrence NO, émis à travers la peau. Le dispositif comporte une chambre de collecte, présentant une ouverture latérale. Le dispositif comporte une chambre de mesure disposée en aval de la chambre de collecte. Il en résulte que le gaz circulant dans la chambre de mesure comporte une part non négligeable d'air externe, ce qui nuit à la sensibilité.

Le document JP2010148692 décrit un dispositif permettant une mesure d'un gaz, en l'occurrence H₂, émis à travers la peau. Le dispositif comporte une ouverture latérale permettant soit l'admission d'un gaz dans une chambre de collecte, soit l'évacuation du gaz. Un dispositif compact permettant la mesure du CO₂ transcutané a déjà été décrit dans WO2020/249466. Il s'agit d'un dispositif de mesure non invasif, porté par un utilisateur, pour estimer une concentration d'un gaz d'intérêt émis de façon transcutanée, le gaz d'intérêt pouvant par exemple être le dioxyde de carbone. La circulation du gaz dans le dispositif permet une collecte du gaz d'intérêt. Le gaz d'intérêt se propage à travers le dispositif par convection, sous l'effet d'une source de chaleur. Le dispositif comporte une face de contact, destinée à être appliquée contre l'utilisateur. Le dispositif comporte également une chambre de mesure, comportant un capteur du gaz d'intérêt, typiquement le CO₂. Pour que l'augmentation de température se traduise par une convection, il est préférable que le la chambre de mesure soit disposée en dessus de la face de contact.

Les inventeurs proposent un perfectionnement du dispositif décrit dans WO2020/249466, visant à améliorer certaines performances, en particulier les performances de réponse temporelle. Les inventeurs proposent également un procédé permettant d'estimer, à l'aide du dispositif, la concentration d'un analyte dans le milieu face auquel le dispositif est disposé.

### EXPOSE DE L'INVENTION

Un objet de l'invention est un procédé d'estimation d'une teneur d'un gaz d'intérêt dans un milieu, à l'aide d'un dispositif de mesure, destiné à être disposé contre le milieu, le dispositif de mesure s'étendant entre une face de contact, destinée à être appliquée contre le milieu et une extrémité distale, le dispositif de mesure comportant une paroi latérale, s'étendant entre la face de contact et l'extrémité distale, le dispositif de mesure comportant :
- au niveau de la face de contact, au moins une ouverture d'admission, configurée pour collecter le gaz d'intérêt émis par le milieu, l'ouverture d'admission étant pratiquée à travers la face de contact;
- une chambre de mesure, comportant un capteur de gaz, le capteur de gaz étant configuré pour mesurer une concentration de gaz d'intérêt s'écoulant à travers la chambre de mesure;
- une chambre de collecte, reliée à la chambre de mesure, et délimitée par une ouverture sur la paroi latérale, la chambre de collecte comportant au moins une ouverture latérale, ménagée à travers la face latérale, ou sur la paroi supérieure de la chambre de collecte de façon à admettre de l'air ambiant dans la chambre de collecte ;
le dispositif étant tel que :
- la chambre de mesure est disposée entre la face de contact et la chambre de collecte;
- le dispositif comporte un moyen d'entraînement, configuré pour entraîner l'air de la chambre de collecte vers une ouverture d'évacuation, l'entraînement d'air induisant un transport du gaz d'intérêt de la face de contact vers la chambre de collecte, à travers la chambre de mesure :
- le procédé comportant :
   - a) mesure de la concentration de gaz d'intérêt dans la chambre de mesure ;
   - b) à l'aide d'une unité de traitement, modélisation du transport du gaz d'intérêt, entre le milieu et la chambre de collecte, la modélisation comportant une prise en compte de la diffusion du gaz depuis le milieu jusqu'à la chambre de collecte à travers la face de contact et le dispositif, ainsi qu'une convection du gaz dans la cellule de collecte résultant de l'entraînement produit par le moyen d'entraînement ;
   - c) à partir de la mesure résultant de l'étape a), et d'une prise en compte du modèle résultant de l'étape b), estimation de la teneur du gaz d'intérêt dans le milieu.

Selon un mode de réalisation,
- le dispositif est spatialement discrétisé selon un maillage spatial, définissant des points de maillage entre la face de contact et la chambre de collecte ;
- le modèle est un modèle spatio-temporel discrétisé, de façon à estimer une teneur en gaz d'intérêt en différents points du maillage, et en différents instants.

Le procédé peut être tel que l'étape b) comporte :
- modélisation de la diffusion du gaz d'intérêt à travers la chambre de mesure ;
- modélisation de la diffusion et de la convection du gaz d'intérêt dans la chambre de collecte.

L'étape b) comporte une modélisation du transport du gaz d'intérêt dans le milieu.

De préférence, l'étape c) met en œuvre un estimateur récursif. Il peut s'agir d'un estimateur récursif linéaire. L'estimateur récursif linéaire peut être un filtre de Kalman.

Selon une application, le milieu est la peau d'un utilisateur, la peau s'étendant en dessus d'un vaisseau sanguin. L'étape b) peut alors comporter :
(i) à partir de la concentration de gaz d'intérêt dans la chambre de mesure, résultant de l'étape a), estimation d'une concentration de gaz d'intérêt transcutané ;
(ii) à partir de la concentration de gaz d'intérêt transcutané résultant de la sous-étape (i), estimation d'une concentration ou pression partielle de gaz d'intérêt dissous dans le sang de l'utilisateur.

Le procédé peut comporter une modélisation du transport du gaz d'intérêt émis par le milieu à travers le dispositif.

Le milieu à analyser peut être un milieu solide ou liquide.

Le moyen d'entraînement peut être un propulseur d'air ou un aspirateur d'air.

La modélisation peut être basée sur un modèle bidimensionnel, le milieu, la chambre de mesure et la chambre de collecte étant discrétisés selon différents points de maillage répartis selon un axe parallèle à la face de contact et un axe perpendiculaire à la face de contact.

L'invention sera mieux comprise à la lecture de l'exposé des exemples de réalisation présentés, dans la suite de la description, en lien avec les figures listées ci-dessous.

### FIGURES

La figure 1A montre un premier mode de réalisation d'un dispositif selon l'invention.
La figure 1B schématise les écoulements à l'intérieur du dispositif.
La figure 2A schématise les principaux composants d'un capteur de gaz, disposé dans la chambre de mesure du dispositif. La vue est prise dans un plan perpendiculaire à un axe transversal.
La figure 2B schématise les principaux composants d'un capteur de gaz, basé sur une absorption d'une lumière infra-rouge, et disposé dans la chambre de mesure du dispositif. La vue est prise dans un plan parallèle à l'axe transversal.
La figure 3 schématise une décomposition du dispositif de mesure et du milieu à analyser en compartiments.
La figure 4 schématise une discrétisation de chaque compartiment représenté sur la figure 3.
La figure 5 illustre les principales étapes de mise en œuvre d'un procédé selon l'invention.
Les figures 6A, 6B et 6C montrent une estimation d'une concentration de CO2 en différents points d'échantillonnage lorsque la concentration dans le sang suit une variation en créneau.
La figure 6D montre une estimation de la concentration de CO2 mesurée par le capteur de gaz du dispositif, ainsi qu'une application d'un bruit blanc sur ladite concentration. L'application du bruit blanc
Les figures 7A, 7B, 7C, 7D montrent une estimation d'une concentration de CO2 en différents points d'échantillonnage lorsque la concentration mesurée par le capteur suit la concentration bruitée simulée sur la figure 6D.
La figure 7E est une comparaison entre la concentration dans le sang en créneau, qui représente la réalité, et la concentration dans le sang estimée par l'estimateur récursif.
La figure 8 schématise une discrétisation de chaque compartiment selon deux dimensions.
La figure 9 illustre une interface qui sépare deux compartiments adjacents.

### EXPOSE DE MODES DE REALISATION PARTICULIERS

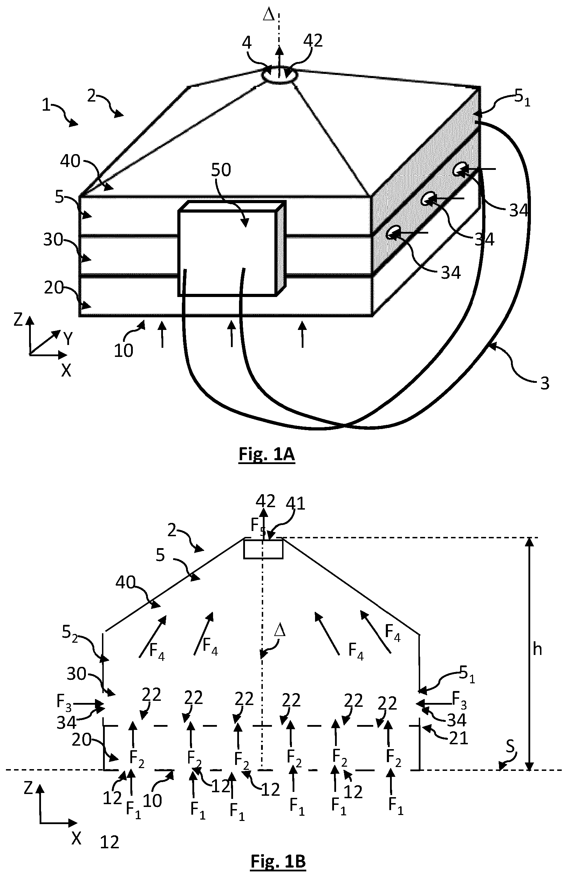

Les figures 1A et 1B sont des vues générales d'un exemple de dispositif 1 selon l'invention. Le dispositif 1 est destiné à être disposé au contact d'un milieu que l'on souhaite analyser. Dans l'exemple décrit, le milieu est de la peau S d'un utilisateur, humain ou animal. Le dispositif comporte un corps principal 2 ainsi qu'un élément de fixation 3, ce dernier étant, dans cet exemple, un bracelet. De façon alternative, le corps principal peut être disposé au contact du lobe d'une oreille, ou d'un doigt. Le support peut être une pince ou être intégré dans un casque auditif. De façon plus générale, le support est configuré pour maintenir le corps principal au contact du milieu à analyser.

Le dispositif est destiné à estimer une concentration d'un gaz d'intérêt émanant de la peau d'un utilisateur. Par gaz d'intérêt, il est entendu un gaz dont on souhaite déterminer une concentration dans un corps humain ou animal vivant, et plus particulièrement dans le sang. Dans l'exemple décrit ci-dessous, de façon non limitative, le gaz d'intérêt est le dioxyde de carbone, dont on cherche à estimer une teneur dans le sang de l'utilisateur. Selon d'autres possibilités, le gaz d'intérêt peut être, de façon non limitative, l'oxygène, l'alcool éthylique, le monoxyde de carbone, le méthane, le monoxyde d'azote, l'acétone ou l'isoprène, l'hydrogène, certaines drogues ou substances volatiles.

Le corps principal 2 comporte une face de contact 10, destinée à être apposée sur la peau S. La face de contact 10 est sensiblement planaire, au sens où elle s'étend parallèlement à un plan XY, certaines portions pouvant être inclinées par rapport au plan XY. Le corps principal comporte également une extrémité distale 4, opposée à la face de contact 10. La surface de contact 10 et l'extrémité distale 4 sont reliées l'une à l'autre par une face latérale 5, s'étendant autour d'un axe central Δ, parallèle à un axe transversal Z, ce dernier étant perpendiculaire au plan XY.

La face de contact 10 peut être formée par une membrane perméable au CO₂ ou une plaque comportant des ouvertures d'admission 12 permettant un passage du CO₂. La face de contact peut comporter une membrane hydrophobe, de façon à éviter la diffusion de la vapeur d'eau à travers le dispositif 1. La face de contact peut être chauffée, ce qui permet d'augmenter le débit sanguin. Cela améliore la collecte.

Selon une possibilité, le dispositif comporte également un élément de chauffage, permettant de porter la face de contact 10, délimitant la chambre de mesure 20, à une température supérieure à 37°C, et de préférence comprise entre 40°C et 50°C, et de préférence entre 40°C et 45 °C, par exemple 42°C. L'élément de chauffage est par exemple une résistance ménagée sur la face de contact, produisant un chauffage par effet Joule. Une augmentation locale et modérée de la température, au voisinage de la peau, favorise en effet une augmentation du débit sanguin par dilatation des capillaires sanguins, ce qui augmente la diffusion d'un gaz d'intérêt transcutané, à travers la peau.

La face de contact 10 débouche sur une chambre de mesure 20. La fonction de la chambre de mesure est d'estimer une concentration de CO₂ du mélange gazeux circulant dans le corps principal 2, parallèlement, ou sensiblement parallèlement, à l'axe central Δ. A cette fin, la chambre de mesure 20 comporte un capteur de gaz 23. Plusieurs types de capteurs peuvent être utilisés à cette fin, par exemple des capteurs optiques ou des capteurs électrochimiques, ces derniers pouvant notamment être basés sur des oxydes métalliques (capteurs MOX), ou des capteurs photoacoustiques. Les inventeurs ont estimé qu'il était préférable d'utiliser un capteur optique, et plus précisément un capteur infra-rouge. Un tel capteur ne nécessite pas de maintenance particulière, et est particulièrement compact, ainsi que peu onéreux. De plus, un tel capteur est très spécifique pour caractériser des liaisons chimiques. Il convient à la détection de molécules de petites tailles, par exemple le dioxyde de carbone.

Le capteur de gaz est un capteur de type NDIR (Non Dispersive Infra Red). Ce type de capteur comporte une source de rayonnement infrarouge 24, émettant généralement dans une bande spectrale comprise entre 1 µm et 20 µm. Il comporte également au moins un photodétecteur de mesure 25, sensible au rayonnement infra-rouge. Le principe repose sur l'atténuation, par le gaz analysé, du rayonnement infra-rouge, émis par la source. La source infra-rouge 24 et le photodétecteur de mesure 25 forment le capteur de gaz 23. Le photodétecteur de mesure 25 est par exemple une thermopile. Un filtre 26 placé devant la thermopile détermine la longueur d'onde du rayonnement infrarouge mesuré par la thermopile. Différentes configurations du capteur de gaz sont décrites par la suite.

Le capteur de gaz 23 est configuré de façon à ce que le gaz d'intérêt transcutané, en l'occurrence le CO₂, se propage entre la source de lumière et le photodétecteur, parallèlement à l'axe central Δ, ou sensiblement parallèlement à l'axe central Δ. Par sensiblement parallèlement, on entend parallèlement en admettant une tolérance angulaire inférieure à ± 30° ou à ± 20° par rapport à la parallèle.

La source infra-rouge émet une lumière se propageant perpendiculairement à l'axe transversal Z. De préférence, la lumière émise par la source est un faisceau en forme de nappe lumineuse perpendiculaire à l'axe transversal Z. Selon l'axe transversal Z, l'épaisseur du faisceau est de préférence inférieure à 1 cm, et de préférence inférieure à 5 mm. De préférence, la nappe lumineuse s'étend sur au moins 50 %, voire au moins 80% de la section transversale de la face de contact 10, cette dernière étant comprise entre quelques cm² et 25 ou 30 cm². Par section transversale, on entend une surface perpendiculaire à l'axe transversal Z (ou à l'axe central Δ). La faible épaisseur du faisceau permet de diminuer le temps de réponse. La surface élevée de la section transversale du faisceau permet d'augmenter la quantité de gaz prélevée et donc la sensibilité de la mesure.

Afin de favoriser un écoulement selon l'axe transversal Z, à travers la chambre de mesure 20 la face de contact comporte une multitudes d'ouvertures d'admission 12, réparties selon une section transversale de quelques cm², par exemple entre 5 et 25 cm². Alternativement, la face de contact est une membrane poreuse, perméable au CO₂. La chambre de mesure 20 débouche sur une chambre de collecte 30. L'interface entre la chambre de mesure 20 et la chambre de collecte 30 peut être formée par une ouverture de collecte large, ou par une pluralité d'ouvertures de collecte 22, ménagées dans une plaque de collecte 21 et réparties selon une section transversale de surface élevée, de façon similaire aux ouvertures d'admission 12. Chaque ouverture de collecte 22 débouche dans une chambre de collecte 30.

La chambre de collecte 30 comporte des ouvertures latérales 34, ménagées dans la face latérale 5 du dispositif 1. Les ouvertures latérales sont configurées pour permettre une admission d'air ambiant à l'intérieur du dispositif 1. L'air ambiant est l'air situé à l'extérieur du dispositif. Le flux d'air ambiant s'écoulant à travers les ouvertures latérales 34 est dirigé vers l'axe central Δ. Chaque ouverture latérale 34 est de préférence orientée perpendiculairement à l'axe central Δ, de façon à ce que l'air ambiant pénètre dans le dispositif 1 , à travers chaque ouverture latérale 34, selon une direction perpendiculaire, ou sensiblement perpendiculaire, à l'axe central Δ.

L'admission de l'air ambiant est obtenue par une pompe 41, agencée pour appliquer une dépression dans la chambre de collecte 30 par rapport à la pression ambiante. La pompe 41 peut être située dans un conduit d'évacuation 40, adjacent à la chambre de collecte 30. Outre l'admission d'air ambiant dans la chambre de collecte, la pompe permet l'écoulement du gaz d'intérêt à travers la chambre de mesure 10 jusqu'à la chambre de collecte 20, à travers les ouvertures de collecte 22. De préférence, le débit est ajusté de façon à ce que l'écoulement du gaz d'intérêt à travers la chambre de mesure 20 soit laminaire. Le débit d'air dans la chambre de collecte peut valoir par exemple entre 0,1 et 10 mL/min, suivant le volume de la chambre.

Dans la chambre de collecte 30, l'air ambiant, admis à travers les ouvertures latérales 34, se mélange avec le gaz d'intérêt, admis à travers les ouvertures de collecte 22. Le conduit d'évacuation 40 est également mis en dépression par la pompe 41, de façon à ce que l'air, mélangé au gaz d'intérêt, est évacué dans le conduit d'évacuation, jusqu'à une ouverture d'évacuation 42, formant l'extrémité distale 4 du dispositif. Dans l'exemple représenté sur les figures 1A et 1B, l'air se propage à travers l'ouverture d'évacuation 42 autour de l'axe central Δ. Selon une autre possibilité, l'air se propage à travers l'ouverture d'évacuation 42 selon un axe d'évacuation sécant, et notamment perpendiculaire, à l'axe central Δ.

Dans l'exemple représenté, les ouvertures latérales sont disposées sur deux faces opposées 5₁, 5₂ de la paroi latérale. Cela favorise une symétrie de l'écoulement d'air dans le dispositif. Toutefois, un tel agencement symétrique des ouvertures latérales n'est pas nécessaire. Les ouvertures latérales 34 peuvent n'être ménagées que sur une face de la paroi latérale, le conduit d'évacuation étant alors disposé sur une face opposée de la paroi latérale.

Chaque ouverture latérale peut être associée à un filtre à CO₂, par exemple un filtre comportant de la chaux, de façon à piéger le CO₂ présent dans l'air ambiant.

Le dispositif comporte une unité de traitement 50. L'unité de traitement 50 comporte des moyens de calcul, par exemple un microprocesseur ou un microcontrôleur, embarqué sur le dispositif, en étant relié par une liaison filaire ou sans fil à un téléphone portable ou à un calculateur de type PC. L'unité de traitement 50 est également configurée pour mettre en œuvre le procédé d'estimation de la teneur en CO₂ dissous du sang de l'utilisateur, à partir de la concentration de CO₂ résultant du capteur de gaz 23.

Les principes généraux régissant l'estimation de la teneur en CO₂ dissous dans le sang à partir de la concentration de CO₂ détectée dans le capteur de gaz ont été décrits dans WO2020/249466.

Le procédé comporte un calcul de la concentration du gaz carbonique dans la chambre de mesure à partir des mesures réalisées par les deux thermopiles à partir de modèles décrivant l'atténuation du rayonnement infrarouge aux deux longueurs d'onde associées à chacune des thermopiles (modèle de Beer Lambert, modèle linéaire quadratique). Ces modèles sont décrits dans WO2020/249466.

La figure 1B schématise les courants fluidiques formés à l'intérieur du dispositif 1. Le CO₂ transcutané est admis dans le dispositif par les ouvertures d'admission 12 pratiquées dans la face de contact 10, et débouchant dans la chambre de mesure 20 (cf. flèche F₁). Le CO₂ se propage à travers la chambre de mesure par diffusion, parallèlement à l'axe central, ce dernier étant parallèle l'axe transversal Z. (cf. flèches F₂). L'air ambiant est admis à travers les ouvertures latérales 34 pratiquées dans la paroi latérale (cf. flèches F₃). Le mélange gazeux comportant l'air ambiant et le CO₂ transcutané est réalisé dans la chambre de collecte 30. Ce mélange se propage vers l'ouverture d'évacuation 42 (cf. flèches F₄) pour sortir du corps 2 du dispositif (cf. flèches F₅)

Un aspect important du dispositif est le recours à la pompe 41, qui permet de former un courant de convection d'air au niveau de la chambre de collecte 30, pour évacuer le mélange air / gaz d'intérêt transcutané. Cela permet également d'accélérer la circulation d'air dans la chambre de collecte. Il en résulte un effet de pompage accélérant le transport du CO₂ du sang vers l'air, à travers la peau et la chambre de mesure. Il en résulte une diminution du temps de réponse du dispositif.

La chambre de mesure 20 comporte, de préférence, un capteur de température ainsi qu'un capteur d'humidité et un capteur de pression.

Le capteur de température sert à convertir la concentration mesurée en pression suivant la loi des gaz parfaits. A l'aide de la loi des gaz parfaits, nous en déduisons la pression partielle du gaz carbonique ${P}_{{CO}_{2}}^{mes} \left({T}^{mes}\right)$ dans la chambre de mesure à la température *T^{mes}* : ${P}_{{CO}_{2}}^{mes} \left({T}^{mes}\right) = {C}_{{CO}_{2}}^{mes} . R . {T}^{mes}$

Où ${C}_{{CO}_{2}}^{mes}$ est exprimée en (mol/m³), *R* est la constante des gaz parfaits (0.0623637 m³.mmHg.K⁻¹.mol ⁻¹), *T^{meas}* en K et ${P}_{{CO}_{2}}^{mes}$ en mmHg.

Le capteur de pression atmosphérique ${P}_{air}^{mes}$ sert à calculer la pression relative ${P}_{{CO}_{2}}^{mes rel}$ exprimée en ppm (partie par million): ${P}_{{CO}_{2}}^{mes rel} = \frac{{P}_{{CO}_{2}}^{mes}}{{P}_{air}^{mes}} {.10}^{6}$

Où ${P}_{{CO}_{2}}^{mes}$ et ${P}_{air}^{mes}$ sont exprimées dans la même unité, ici en mmHg.

Le capteur d'humidité sert à calculer la concentration de la vapeur d'eau dans la chambre de mesure. Un facteur d'atténuation du rayonnement important concerne la vapeur d'eau. La prise en compte de l'humidité nécessite de convertir la valeur d'humidité relative *RH^{mes}* fournie par le capteur d'humidité en concentration de vapeur d'eau ${C}_{{H}_{2} O}^{mes}$. L'humidité relative correspond à la pression partielle d'eau dans la chambre de mesure, notée ${P}_{{H}_{2} O}^{mes} \left({T}^{mes}\right)$*,* sur la pression de vapeur saturante à la température *T^{mes}* de la chambre de mesure. ${RH}^{mes} ≜ \frac{{P}_{{H}_{2} O}^{mes}}{{VP}_{H 2 O} \left({T}^{mes}\right)} .100 %$

La concentration en H₂O dans la chambre de mesure peut être obtenue selon l'expression : ${P}_{{H}_{2} O}^{mes} = \frac{{n}_{{H}_{2} O}^{mes}}{{V}^{mes}} R {T}^{mes} = {C}_{{H}_{2} O}^{mes} . R . {T}^{mes}$

En combinant les deux équations précédentes, on obtient : ${C}_{{H}_{2} O}^{mes} = \frac{{RH}^{mes} . {VP}_{H 2 O} \left({T}^{mes}\right)}{100 % R . {T}^{mes}}$

Où VP_{H2O}(*T^{mes}*) représente les valeurs de pression de vapeur d'eau saturante En fonction de la température ces valeurs peuvent être obtenues à partir de tables, où à partir d'une expression analytique, par exemple l'équation de Tetens ${VP}_{H 2 O} \left({T}^{mes}\right) = 0.61078 {e}^{\left(\frac{17.27 \left({T}^{mes}-273.15\right)}{{T}^{mes} - 35.85}\right)}$

La concentration ${C}_{{H}_{2} O}^{mes}$ peut être utilisée pour estimer la concentration de CO₂ dans la chambre de mesure, en utilisant l'expression de Beer-Lambert $\begin{matrix}- ln \left[\frac{U \left({λ}_{1}\right) / U \left({λ}_{2}\right)}{{U}_{0} \left({λ}_{1}\right) / {U}_{0} \left({λ}_{2}\right)}\right] \\ = \left[{k}_{{CO}_{2}}\left({λ}_{1}\right)-{k}_{{CO}_{2}}\left({λ}_{2}\right)\right] . {C}_{{CO}_{2}} + \left[{k}_{{H}_{2} O}\left({λ}_{1}\right)-{k}_{{H}_{2} O}\left({λ}_{2}\right)\right] \\ + \left[{A}_{air}\left({λ}_{1}\right)-{A}_{air}\left({λ}_{2}\right)\right]\end{matrix}$

Où *C*_{CO2} et *C*_{*H*2*O*} sont les concentrations molaires, *k*_{CO2} (*λ*₁), *k*_{CO2} (*λ*₂), *k*_{*H*2*O*}(*λ*₁), *k*_{*H*2*O*}(*λ*₂) les coefficients d'atténuation du gaz carbonique et de la vapeur d'eau, *Aₐᵢᵣ*(*λ*₁), *Aₐᵢᵣ*(*λ*₂) les atténuations de l'air aux longueurs d'onde *λ*₁, *λ*₂.

Ainsi, la prise en compte de la concentration d'H₂O permet d'améliorer la précision avec laquelle la concentration de CO₂ est mesurée, comme évoqué dans la demande de brevet WO2020/249466.

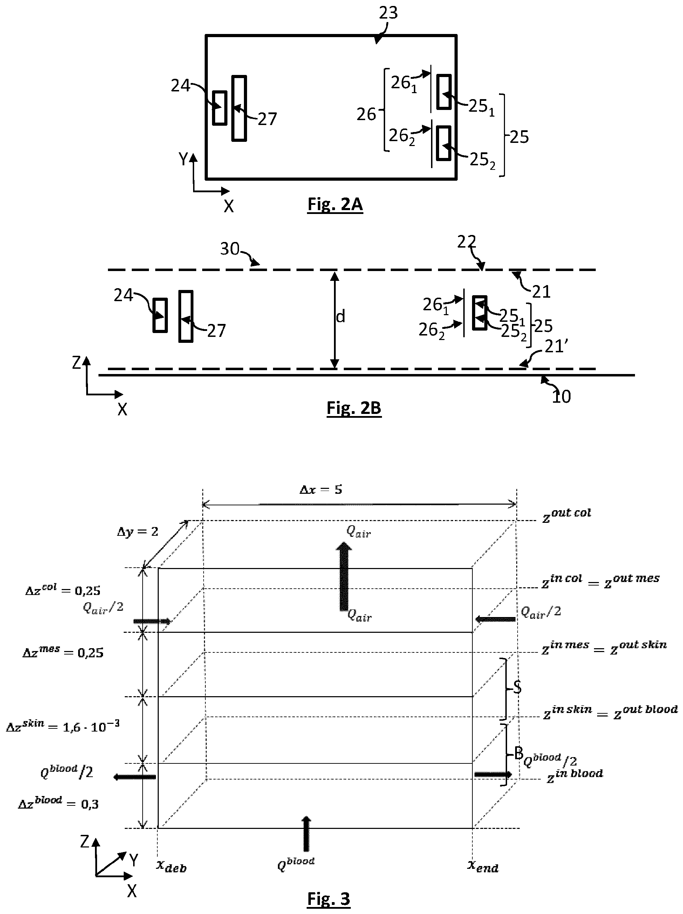

La figure 2B montre la chambre de mesure dans un plan P_{XZ} parallèle à l'axe transversal Z. La chambre de mesure peut s'étendre entre deux grilles 21 et 21' perméables au gaz d'intérêt. La grille 21 forme la paroi d'interface avec la chambre de collecte 30. Elle comporte des ouvertures de collecte, permettant l'écoulement du gaz d'intérêt de la chambre de mesure vers la chambre de collecte. De préférence, chaque grille est réfléchissante dans la bande spectrale d'émission de la source infra-rouge. La chambre de mesure peut comporter une grille réfléchissante 21', similaire à la grille 21, au niveau de la face de contact 10. La présence de chaque grille réfléchissante permet d'augmenter la quantité de lumière se propageant à travers le gaz d'intérêt, vers chaque voie du photodétecteur. Elle permet également de limiter l'épaisseur de la nappe de lumière, selon l'axe transversal.

La distance d entre la grille 21 et la face de contact, ou entre les grilles 21 et 21', est par exemple comprise entre 3 mm et 8 mm.

Les mesures résultant du capteur peuvent être bruitées, auquel cas un filtrage passe bas peut être appliqué. Le filtrage passe-bas peut être réalisé par exemple par un filtre à moyenne glissante dont la longueur est adaptée au niveau du bruit de mesure.

A partir de ${C}_{CO 2}^{mes}$*,* la pression partielle de CO2 ${P}_{CO 2}^{mes} \left({T}^{mes}\right)$ est déduite par : ${P}_{CO 2}^{mes} \left({T}^{mes}\right) = {C}_{CO 2}^{mes} . R . {T}^{mes}$

A partir du capteur de pression atmosphérique de la chambre de mesure, la pression relative ${P}_{CO 2}^{mes , rel} \left({T}^{mes}\right)$ est calculée par : ${P}_{CO 2}^{mes , rel} \left({T}^{mes}\right) = \frac{{P}_{CO 2}^{mes} \left({T}^{mes}\right)}{{P}_{air}^{mes}} {10}^{6}$

Où ${P}_{air}^{mes}$ est la pression atmosphérique dans la chambre de mesure.

A partir de la concentration de CO2 résultant du capteur de mesure, la concentration en gaz carbonique dans le sang peut être estimée en appliquant un modèle du transport de CO2 entre le sang et la chambre de mesure. On décrit par la suite un modèle de transport monodirectionnel, modélisant le transport de CO2, par diffusion et par convection.

### Premier modèle : Modèle unidimensionel 1D

La figure 3 schématise un exemple de dispositif apposé sur la peau S de l'utilisateur. Le sang occupe un compartiment sang B, entre les coordonnées *z^{in blood}* et *z^{out blood}*, et son épaisseur est Δ*z^{blood}.* Le compartiment sang B est recouvert par de la peau S. La peau S s'étend entre les coordonnées *z^{in skin}* et *z^{out skin},* et son épaisseur est Δ*z^{skin}. z^{in skin}* = *z^{out blood}* . La peau est recouverte par le dispositif. Dans le dispositif de l'art antérieur, le dispositif comporte une superposition de la chambre de collecte et de la chambre de mesure. La chambre de collecte s'étend entre les coordonnées *z^{in col}* et *z^{out col}*, et son épaisseur est Δ*z^{col}.* La chambre de mesure s'étend entre les coordonnées *z^{in mes}* et *z^{out mes},* et son épaisseur est Δ*z^{mes}*. *z^{in col}* = *z^{out peau}* ; *z^{in mes}* = *z^{out col}.* Selon l'invention, le dispositif comporte une superposition de la chambre de mesure 20, de la chambre de collecte 30.

La diffusion est caractérisée par un coefficient de diffusion, qui dépend du milieu traversé, en l'occurrence le sang, la peau et l'air.

Dans la modélisation 1D, le transport du gaz carbonique est décrit uniquement selon l'axe central Δ correspondant à l'axe Z. Le sang s'écoule, dans le compartiment sang, selon un courant de convection de vitesse moyenne ${u}_{z}^{blood}$. Dans la peau, le CO₂ migre uniquement par diffusion. Dans le dispositif, dans la cellule de mesure le CO₂ migre uniquement par diffusion et dans la cellule de collecte le CO₂ migre par diffusion et par convection. La convection résulte de la circulation d'air induite par la pompe dans la chambre de collecte. La convection d'air dans la chambre de collecte entraîne une diffusion à travers la face de contact et à travers la chambre de mesure. Elle est caractérisée par une vitesse moyenne ${u}_{z}^{col}$ selon l'axe Z.

En se basant sur une propagation du gaz d'intérêt selon une direction Z perpendiculaire à la surface de la peau (modèle monodimensionnel), on peut écrire dans tout milieu homogène (_i-e de même constante de Henry) : $\frac{\partial C \left(z, t\right)}{\partial t} = \frac{\partial }{\partial z} \left[-u\left(z\right)C\left(t, z\right)\right] + \frac{\partial }{\partial z} \left[D\left(z\right)\frac{\partial C \left(t, z\right)}{\partial z}\right] + R \left(z, t\right)$ où :
- u(z) est la vitesse de propagation du CO2 à une coordonnée z selon l'axe Z ;
   - *C*(*t, z*) est la concentration du CO2 à l'instant t et à la coordonnée z ;
   - D(z) est le coefficient de diffusion du CO2 selon la direction de propagation Z, à la coordonnée z.
   - *R*(*z, t*) correspond à la sortie du CO2 dans le sang au niveau de la peau (terme puit) ainsi qu'à l'arrivée du CO₂ par les parois latérales dans la chambre de collecte (terme source). *R*(*z, t*) est donc non nul uniquement au niveau de l'interface sang/peau et de l'interface chambre de mesure/chambre de collecte à l'intérieur du dispositif.

En considérant un milieu homogène : $\frac{\partial }{\partial z} D \left(z\right) = 0$ *et* $\frac{\partial }{\partial z} u \left(z\right) = 0$

L'équation (10) devient $\frac{\partial C \left(z, t\right)}{\partial t} = - {u}_{z} \frac{\partial C \left(z, t\right)}{\partial z} + D \frac{{\partial }^{2} C \left(z, t\right)}{\partial {z}^{2}} + R \left(z, t\right)$

Dans l'expression (11), le terme $- {u}_{z} \frac{\partial C \left(z, t\right)}{\partial z}$ correspond à la dynamique due à la convection. Le terme $D \frac{{\partial }^{2} C \left(z, t\right)}{\partial {z}^{2}}$ correspond à la dynamique due à la diffusion. L'expression (11) correspond à une équation de convection - diffusion.

L'expression (11) modélise le transport du gaz d'intérêt selon l'axe Z à l'intérieur de chaque compartiment précédemment décrit (chambre de mesure, chambre de collecte).

Le modèle prend également en compte une équation d'observation :
*C*(*zₘₑₛ, t*) *= y* (12) où y correspond à la concentration mesurée ${C}_{{CO}_{2}}^{mes}$ dans la cellule de mesure et *zₘₑₛ* est la coordonnée spatiale selon l'axe Z du point où est réalisée la mesure.

Les conditions aux limites sont : $C \left({z}_{blood 0}, t\right) = {C}_{in blood} \left(t\right)$ où *z*_{*blood* 0} correspond à la coordonnée, selon Z, de l'entrée du compartiment sang. et $\frac{\partial C \left({z}_{col}, t\right)}{\partial z} = 0$ où *z_{col}* correspond à la coordonnée, selon Z, de l'extrémité distale du dispositif.

On décrit à présent les conditions aux interfaces. Dans les équations qui suivent, les indices i et j désignent deux milieux successifs respectivement en amont et en aval de l'interface, l'amont et l'aval étant pris en compte dans le sens de propagation nette du CO2, c'est-à-dire orienté du compartiment sang vers la chambre de collecte.

Considérant que *z*_{*i*|*j*} représente la coordonnée spatiale selon l'axe Z de l'interface entre le milieu i et le milieu j, *Jᵢ*(*z*_{*i*|}*ⱼ, t*) est le flux gazeux sortant du milieu i et *Jⱼ*(*z*_{*i*|}*ⱼ, t*) est le flux gazeux entrant dans le milieu j, par unité de surface. Pour une interface transparent au gaz nous avons : ${J}_{i} \left({z}_{\left.i\right| j}, t\right) = {J}_{j} \left({z}_{\left.i\right| j}, t\right)$ avec : ${J}_{i} \left({z}_{\left.i\right| j}, t\right) = {u}_{i} \left({z}_{\left.i\right| j}\right) C \left({z}_{\left.i\right| j}, t\right) - {D}_{i} \left({z}_{\left.i\right| j}\right) ∇ C \left({z}_{\left.i\right| j}, t\right)$ et ${J}_{j} \left({z}_{\left.i\right| j}, t\right) = {u}_{j} \left({z}_{\left.i\right| j}\right) C \left({z}_{\left.i\right| j}, t\right) - {D}_{j} \left({z}_{\left.i\right| j}\right) ∇ C \left({z}_{\left.i\right| j}, t\right)$

Dans les expressions (16) et (17), *uᵢ*(*z*_{*i*|*j*})*C*(*z*_{*i*|}*ⱼ, t*) et *uⱼ*(*z*_{*i*|*j*})*C*(*z*_{*i*|}*ⱼ, t*) correspondent à la convection, tandis que *Dᵢ*(*z*_{*i*|*j*})∇*C*(*z*_{*i*|}*ⱼ, t*) et *Dⱼ*(*z*_{*i*|*j*})∇*C*(*z*_{*i*|}*ⱼ, t*) correspondent à la diffusion.

On considère qu'aux interfaces entre le sang et la peau, ou entre la peau et la chambre de mesure, ou entre la chambre de mesure et la chambre de collecte, la vitesse est nulle de part et d'autre de l'interface : *uᵢ*(*z*_{*i*|*j*}) *= uⱼ*(*z*_{*i*|*j*}) = 0. En effet, la convection n'intervient que dans la chambre de collecte. Dans la peau et dans la chambre de mesure, le CO2 se propage uniquement par diffusion.

En combinant (16) et (17), du fait de la continuité du flux, et en introduisant un facteur de transparence, on obtient alors : ${r}_{transpa \left. i\right| j} {D}_{i} \frac{\partial {C}_{i} \left({z}_{\left.i\right| j}, t\right)}{\partial z} = {D}_{j} \frac{\partial {C}_{j} \left({z}_{\left.i\right| j}, t\right)}{\partial z}$

Où *r*_{*transpa i*|*j*} correspond au facteur de transparence entre le compartiment i et le compartiment j. Le facteur de transparence correspond à un ratio entre la surface ouverte de l'interface, laissant passer le gaz d'intérêt, sur la surface totale de l'interface.

De part et d'autre de l'interface, la pression est identique : ${P}_{i} \left({z}_{\left.i\right| j}, t\right) = {P}_{j} \left({z}_{\left.i\right| j}, t\right)$

Or ${C}_{i} \left({z}_{\left.i\right| j}, t\right) = \frac{{H}_{i}}{{RT}_{i}} {P}_{i} \left({z}_{\left.i\right| j}, t\right)$

Où *Tᵢ* est la température du milieu i, et *Tᵢ = Tⱼ* $\frac{{C}_{i} \left({z}_{\left.i\right| j}, t\right)}{{H}_{i}} = \frac{{C}_{j} \left({z}_{\left.i\right| j}, t\right)}{{H}_{j}} {=}^{def} {C}_{\left.i\right| j} \left({z}_{\left.i\right| j}, t\right)$

Le symbole signifie « égalité qui sert de définition».

### Prise en compte de l'écoulement du sang dans le compartiment sang

Dans le compartiment sang B : , $R \left({z}^{blood 3}, t\right) = - \frac{2 {u}_{x}^{blood}}{Δ x} {C}^{out}$
- Δ*x* est la largeur de la fenêtre d'entrée
- *C^{out}* est la concentration de CO₂ sortant du compartiment sanguin sous l'effet de l'écoulement du sang ;
- ${u}_{x}^{blood}$ est la vitesse d'écoulement du sang selon une direction perpendiculaire à l'axe Z.
- *R*(***z*^{*blood* 3}***, t*) correspond à un terme puit. ${u}_{x}^{blood} = \frac{\frac{{Q}^{blood}}{2}}{{A}_{x}^{out blood}}$

*Q^{blood}* est le débit sanguin et ${A}_{x}^{out blood}$ est la surface de sortie à gauche et à droite du compartiment sang. Il s'agit d'une section des vaisseaux sanguins.

### Prise en compte de l'admission d'air dans la chambre de collecte.

Dans la chambre de collecte, l'admission d'air se traduit par un terme source correspondant au gaz carbonique présent dans l'air ambiant $R \left({z}^{col 1}, t\right) = \frac{2 {u}_{x}^{col}}{Δ x} {C}^{in col air}$
- Δ*x* correspond à la largeur du dispositif
- ${u}_{x}^{col}$ est le courant d'arrivée d'air
- *C^{in col air}* est la concentration d'air qui pénètre dans la chambre de collecte ;
- *R*(***z*^{*col* 1}***, t*) correspond à un terme source. ${u}_{x}^{col} = \frac{\frac{{Q}^{air}}{2}}{{A}_{x}^{in col}}$

*Q^{air}* est le débit d'air et ${A}_{x}^{in col}$ est la surface d'entrée le long des parois latérales de la cellule de collecte.

### Discrétisation du modèle.

Le dispositif et le milieu sur lequel il est appliqué ont été discrétisés

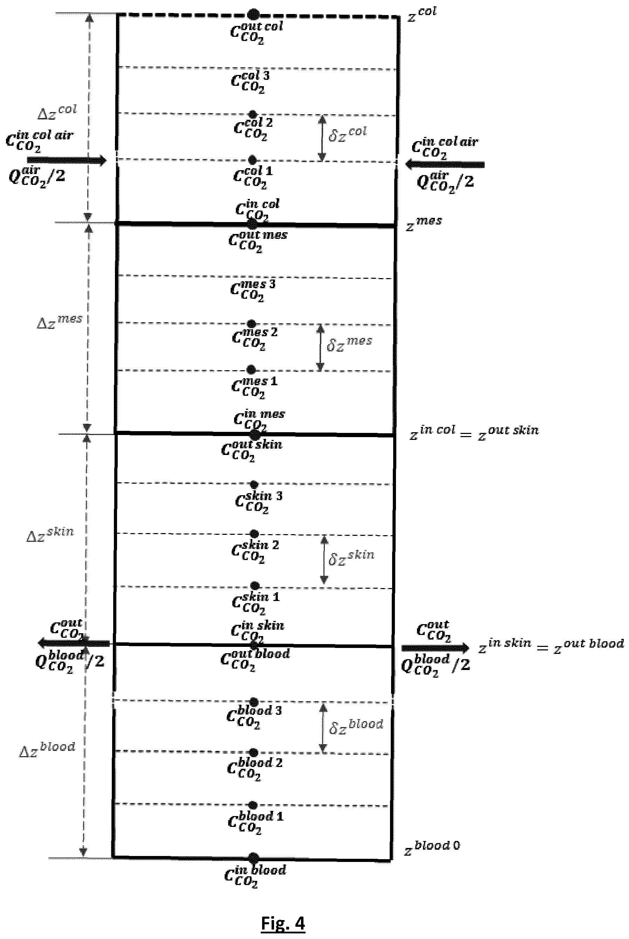

L'équation de convection - diffusion (11) peut être discrétisée spatialement comme représenté sur la figure 4. Un maillage spatial a été réalisé, générant 17 points d'échantillonnage équirépartis dans chaque compartiment, entre le compartiment sang B et la chambre de collecte. Chaque compartiment comporte 5 points de d'échantillonnage, dont 2 sont placés au niveau d'une interface inférieure ou supérieure. Au niveau d'une interface entre un compartiment aval et un compartiment amont, la concentration sortant dans le compartiment aval est référencée par un exposant « out » tandis que la concentration entrant dans le compartiment amont est référencée par un exposant « in ».

La concentration à l'intérieur du compartiment sang est discrétisée selon 3 points d'échantillonnage : ${C}_{CO 2}^{blood , 1} ; {C}_{CO 2}^{blood , 2} ; {C}_{CO 2}^{blood , 3}$

La concentration à l'intérieur du compartiment peau est discrétisée selon 3 points d'échantillonnage: ${C}_{CO 2}^{skin , 1} ; {C}_{CO 2}^{skin , 2} ; {C}_{CO 2}^{skin , 3}$

La concentration à l'intérieur de la chambre de mesure est discrétisée selon 3 points d'échantillonnage: ${C}_{CO 2}^{mes , 1} ; {C}_{CO 2}^{mes , 2} ; {C}_{CO 2}^{mes , 3}$

La concentration à l'intérieur et en sortie de la chambre de collecte est discrétisée selon 4 points d'échantillonnage: ${C}_{CO 2}^{col , 1} ; {C}_{CO 2}^{col , 2} ; {C}_{CO 2}^{col , 3} ; {C}_{CO 2}^{out col}$

Au niveau des interfaces entre les compartiments, les concentrations sont

Nous définissons des concentrations de CO2 aux interfaces :
- Au niveau de l'interface sang / peau : $\frac{{C}_{CO 2}^{out blood}}{{H}^{blood}} = \frac{{C}_{CO 2}^{in skin}}{{H}^{skin}}$;
   *H^{blood}* et *H^{skin}* correspondent aux constantes de Henry du sang et de la peau.
- Au niveau de l'interface peau/cellule de mesure $\frac{{C}_{CO 2}^{out skin}}{{H}^{skin}} = \frac{{C}_{CO 2}^{in mes}}{{H}^{air}}$
   *H^{air}* correspond à la constante de Henry de l'air.
- Au niveau de l'interface cellule de mesure / cellule de collecte ${C}_{CO 2}^{out mes} = {C}_{CO 2}^{in col}$

La concentration d'entrée est ${C}_{CO 2}^{in blood}$. Dans la formulation du problème direct, ${C}_{CO 2}^{in blood}$ est connu. Lorsqu'on résout le problème inverse, c'est ${C}_{CO 2}^{in blood}$ qui est recherché.

L'écart spatial *δz* entre chaque point du maillage d'un même compartiment est identique. Par la suite, l'écart entre chaque point du maillage est noté *δzᵢ* , l'indice i désignant le milieu : i = 1 pour le sang, i = 2 pour la peau, i = 3 pour la chambre de mesure et i = 4 pour la chambre de collecte.

Compte tenu de la discrétisation spatiale effectuée, l'équation de convection diffusion (expression 11) devient : $\begin{matrix}\frac{C \left(z,t+δt\right) - C \left(z, t\right)}{δt} \\ = \left[\frac{{uδz}_{i} + 2 D}{2 {\left({δz}_{i}\right)}^{2}}\right] C \left(z-{δz}_{i},τ\right) + \left[\frac{- 2 D}{{\left({δz}_{i}\right)}^{2}}\right] C \left(z, τ\right) \\ + \left[\frac{- {uδz}_{i} + 2 D}{2 {\left({δz}_{i}\right)}^{2}}\right] C \left(z+{δz}_{i},τ\right)\end{matrix}$

Dans cet exemple, *τ = t* + *δt* : on utilise un schéma d'intégration dit implicite.

On définit un vecteur ***z***, de dimension (17, 1), qui comporte l'ensemble des points de maillage. $z = {\left({z}_{i}\right)}_{i = 1}^{n}$

Le nombre total d'échantillons est *N*, tel que $N = {\sum }_{i = 1}^{4} {N}_{i}$ où *Nᵢ* est le nombre d'échantillons au sein du compartiment i. ${N}_{i} = \frac{Δ {z}_{i}}{{δz}_{i}} - 1$

Où Δ*zᵢ* est la hauteur du milieu *i* et la distance entre deux points successifs dans le milieu *i* est noté *δzᵢ*.

La position suivant z dans la colonne, à laquelle commence chaque milieu, est définie par *z_{deb i}.* Cette position peut être calculée de manière générale par les relations suivantes: ${z}_{deb i} = \left\{\begin{matrix}{z}_{0} , pour i = 1 \\ {z}_{0} + {\sum }_{j = 1}^{i - 1} Δ {z}_{i} , pour i \geq 2\end{matrix}\right.$

### Modèle direct

On définit un vecteur c par rapport aux concentrations évaluées dans différents points d'échantillonnage définis par le vecteur z.

***c*₀** est un vecteur de dimension (17, 1). Il est obtenu par concaténation des termes des vecteurs ${c}_{i}^{t} \left(t\right)$ avec i compris entre 1 et 4. ${c}_{0} = \left[\begin{matrix}c \left({z}_{deb 1}+k.{δz}_{1}\right) , k = 1 , … , {N}_{1} \\ c \left({z}_{deb 2}+k.{δz}_{2}\right) , k = 1 , … , {N}_{2} \\ c \left({z}_{deb 3}+k.{δz}_{3}\right) , k = 1 , … , {N}_{3} \\ c \left({z}_{deb 4}+k.{δz}_{4}\right) , k = 1 , … , \left({N}_{4}+1\right)\end{matrix}\right]$ ${c}_{0} = \left[\begin{matrix}C \left({z}_{deb 1}+1.{δz}_{1}\right) \\ ⋯ \\ C \left({z}_{deb 1}+{N}_{1}.{δz}_{1}\right) \\ C \left({z}_{deb 2}+1.{δz}_{2}\right) \\ … \\ C \left({z}_{deb 2}+{N}_{2}.{δz}_{2}\right) \\ C \left({z}_{deb 3}+1.{δz}_{3}\right) \\ … \\ C \left({z}_{deb 3}+{N}_{3}.{δz}_{3}\right) \\ C \left({z}_{deb 4}+1.{δz}_{4}\right) \\ … \\ C \left({z}_{deb 4}+\left({N}_{4}+1\right).{δz}_{4}\right)\end{matrix}\right]$

Dans cet exemple, on suppose que le CO2 est mesuré en sortie de la chambre de mesure : $y = C \left({z}_{deb 3}+{N}_{3} {δz}_{3}\right)$

*Nₑₜₐₜ* regroupe les points au bord supérieur et à l'intérieur strict des milieux en ne tenant pas compte des 3 points qui correspondent aux interfaces sang/peau, peau/mes, mes/col ${N}_{etat} = N - 3$

Dans notre cas, nous avons : *N*₁ = *N*₂ *= N*₃ *= N*₄ = 3 donc *N =* 16 et *Nₑₜₐₜ* = 13

On définit un vecteur c par rapport aux concentrations évaluées aux différents points d'état.

On définit un vecteur d'état augmenté ***c̃*** de dimension (*Nₑₜₐₜ +* 1) en ajoutant, au vecteur c la concentration *C^{in blood}* en entrée du milieu sanguin:
L'expression (30) peut être écrite de façon matricielle : $c \left(t+δt\right) - c \left(t\right) = δt ⋅ F ⋅ c \left(τ\right)$
***c***(*t*) et ***c***(*t* + *δt*) sont des vecteurs de dimension (13, 1).Chaque terme de ces vecteurs correspond à une concentration en un point de mesure représenté sur la figure 4, respectivement à l'instant *t* et à l'instant *t + δt*
F est une matrice de passage, de dimension (13,13), telle que $F = {F}_{conv} + {F}_{dif} + {F}_{int} + {F}_{puit}$ Où :
   ***F_{conv}*** regroupe les discrétisations liées à la convection : ***F_{conv}*** de dimension [*Nₑₜₐₜ, Nₑₜₐₜ*] (*Nₑₜₐₜ* = 13 dans cet exemple) est calculée de la manière suivante sur les termes de rang (*q, q +* 1) ${f}_{q , q + 1} = - \frac{{u}_{i}}{2 {δz}_{i}}$

Et sur les termes de rang (*q, q* - 1) : ${f}_{q , q - 1} = \frac{{u}_{i}}{2 {δz}_{i}}$

En dehors des points de rang (q,q+1) et (q, q-1), les termes de ***F_{conv}*** ont une valeur nulle.
***F_{dif}*** regroupe les discrétisations liées à la diffusion ;
***Fᵢₙₜ*** regroupe les discrétisations liées aux interfaces et aux bords ;
et ***Fₚᵤᵢₜ*** contient le terme puit (i-e la sortie du sang au niveau de la peau dans le milieu sanguin).
*uᵢ* correspond à la vitesse de propagation dans la direction z pour le compartiment *i* et *δzᵢ* est la distance entre deux points adjacents d'échantillonnage du même compartiment *i*
***F_{dif}*** de dimension [*Nₑₜₐₜ, Nₑₜₐₜ*] (*Nₑₜₐₜ =* 13 dans cet exemple) est calculée de la manière suivante en ayant :
   - sur la diagonale principale (ligne et colonne de même rang *q*) : ${f}_{q , q} = \frac{- 2 {D}_{i}}{{\left({δz}_{i}\right)}^{2}}$
   - au-dessus de la diagonale principale ${f}_{q , q + 1} = \frac{{D}_{i}}{{\left({δz}_{i}\right)}^{2}}$
   - en-dessous de la diagonale principale ${f}_{q , q - 1} = \frac{{D}_{i}}{{\left({δz}_{i}\right)}^{2}}$

En dehors des points de rang (q,q+1) et (q, q-1), (q,q), les termes de ***F_{dif}*** ont une valeur nulle.

La matrice ***Fᵢₙₜ,*** décrivant les conditions aux interfaces entre deux compartiments adjacents, de dimension [*Nₑₜₐₜ, Nₑₜₐₜ*] (*Nₑₜₐₜ* = 13 dans cet exemple), est calculée comme suit :
Tous les termes de la matrice ***Fᵢₙₜ*** sont nuls, sauf :
- Les termes [1,1] et [1,2], qui correspondent au bord inférieur, à la coordonnée *z*^{*blood* 1} = z_{deb1} + 1.
- Les termes [(z_{deb1} + N₁ · δz₁, z_{deb1} + N₁ · δz₁),(z_{deb1} + N₁ · δz₁, z_{deb2} + δz₂)] et [(z_{deb2} + δz₂, z_{deb1} + N₁ · δz₁),( z_{deb2} + δz₂, z_{deb2} + δz₂)] qui correspondent à la première interface ;
- Les termes [(z_{deb2} + N₂ · δz₂, z_{deb2} + N₂ · δz₂),( z_{deb2} + N₂ · δz₂, z_{deb3} + δz₃)] et [(z_{deb3} + δz₃, z_{deb2} + N₂ · δz₂),( z_{deb3} + δz₃, z_{deb3} + δz₃)] qui correspondent à la deuxième interface ;
- Les termes [(z_{deb3} + N₃ · δz₃, z_{deb3} + N₃ · δz₃),( z_{deb3} + N₃ · δz₃, z_{deb4} + δz₄)] et [( z_{deb4} + δz₄, z_{deb3} + N₃ · δz₃),(z_{deb4} + δz₄, z_{deb4} + δz₄)] qui correspondent à la troisième interface ;
- Les termes (*z*_{*deb*4} + (*N*₄ *+* 1) · *δz*₄, *z*_{*deb*4} *+ N*₄ · *δz*₄), (*z*_{*deb*4} + (*N*₄ + 1) · *δz*₄, *z*_{*deb*4} *+* (*N*₄ *+* 1) · *δz*₄) sont égaux à correspondent au bord supérieur.

Les termes de la matrice différents de ceux précédemment explicités sont égaux à 0. Chaque terme non nul *f_{q,q}* de la matrice ***Fᵢₙₜ*** est tel que : ${f}_{q , q} = \frac{{u}_{i} {δz}_{i} + 2 {D}_{i}}{2 {\left({δz}_{i}\right)}^{2}}$ avec *[q, q]* = [*z*_{*deb*1} + *N*₁ · *δz*₁, *z*_{*deb*1} + *N*₁ · *δz*₁], [*z*_{*deb*2} + *N*₂ · *δz*₂, *z*_{*deb*2} + *N*₂ · *δz*₂], [*z*_{*deb*3} + *N*₃ · *δz*₃, *z*_{*deb*3} + *N*₃ · *δz*₃].

Chaque terme non nul *f_{q,q+2}* de la matrice ***Fᵢₙₜ*** est tel que : ${f}_{q , q + 2} = {λ}_{\left.i\right| j} \frac{{D}_{j}}{{δz}_{j}}$ avec *[q, q + 2]* = [*z*_{*deb*1} + *N*₁ · *δz*₁*, z*_{*deb*2} + *δz*₂], [*z*_{*deb*2} + *N*₂ · *δz*₂, *z*_{*deb*3} + *δz*₃], [*z*_{*deb*3} + *N*₃ · *δz*₃, *z*_{*deb*4} + *δz*₄]
et ${λ}_{\left.j\right| i} = \left[\frac{{u}_{j} {δz}_{j} + 2 {D}_{j}}{2 {\left({δz}_{j}\right)}^{2}}\right] {H}_{j} {ζ}_{\left.i\right| j}$ et ${ζ}_{\left.i\right| j} = \frac{1}{{r}_{transpa \left. i\right| j} {H}_{i} \frac{{D}_{i}}{{δz}_{i}} + {H}_{j} \frac{{D}_{j}}{{δz}_{j}}}$

*Hⱼ* et *Hᵢ* sont les coefficients de Henry respectifs des milieux *i* et *j* et *r*_{*transpa i*|*j*} est le facteur de transparence entre les milieux i et j
La matrice ***Fₚᵤᵢₜ**,* décrivant les conditions aux interfaces entre deux compartiments adjacents, de dimension [*Nₑₜₐₜ, Nₑₜₐₜ*] (*Nₑₜₐₜ* = 13 dans cet exemple), est calculée comme suit. Le seul terme non nul est le terme situé à la position (3, 2) correspondant à l'interface entre le sang et la peau ${F}_{puit} = \left(\begin{matrix} & \begin{matrix}0 & ⋯ & 0 \\ ⋮ & ⋯ & ⋮\end{matrix} & \\ 0 & \frac{- {u}_{z}^{blood}}{2 {δz}^{blood}} & 0 \\ & \begin{matrix}⋮ & ⋯ & ⋮ \\ 0 & ⋯ & 0\end{matrix} & \end{matrix}\right)$

Le terme $\frac{- {u}_{z}^{blood}}{2 {δz}^{blood}}$ correspond à la sortie par convection du compartiment du sang

L'arrivée d'air dans la cellule de collecte est prise en compte dans la matrice de commande décrite par la suite.

### Application d'un estimateur récursif.

Dans cet exemple, on met en œuvre un estimateur récursif pour estimer la concentration de CO2 dans le sang, en fonction du temps. Il s'agit ici d'un estimateur récursif linéaire, de type filtre de Kalman.

L'estimateur est basé sur le modèle d'espace état spatio-temporel précédemment décrit, auquel on ajoute une équation d'observation, qui traduit la relation entre chaque mesure et les états.

De façon connue avec ce type d'estimateur, la concentration, à un instant, est calculée de manière récursive, à partir d'une estimée de ladite concentration, résultant d'une itération précédente, et d'une mesure effectuée à l'instant t.

En partant de l'équation (36) : $C \left(z,t+δt\right) - C \left(z, t\right) = δt ⋅ F ⋅ C \left(z,t+δt\right) ;$

On peut écrire : ${{\left(I-δt⋅F\right)}_{︸}}_{A} C \left(z,t+δt\right) = C \left(z, t\right)$

Par la suite, on utilise les notations suivantes :
- ***c̃*_{*k,k*-1}** : vecteur d'état à l'instant *k* prédit à l'instant *k -* 1 précédent : dimension (*Nₑₜₐₜ*+1) *;*
- ***c̃_{k,k}*** : vecteur d'état à l'instant *k* calculé à l'instant *k* : dimension (*Nₑₜₐₜ*+1) ;
- ***qₖ*** : vecteur de commande, de dimension (Q, 1). Dans cet exemple, Q = 1.
- ***c̃*_{*k*+1*,k*}** : vecteur d'état à l'instant *k +* 1 prédit à l'instant *k* : dimension (*Nₑₜₐₜ*+1) ;
- ***P*_{*k,k*-1}** : matrice de covariance de l'erreur à l'instant *k* prédite à l'instant *k -* 1 précédent : dimension (*Nₑₜₐₜ*+1, *Nₑₜₐₜ*+1);
- ***P_{k,k}*** : la matrice de covariance de l'erreur à l'instant *k* calculée à l'instant *k* : dimension (*Nₑₜₐₜ*+1, *Nₑₜₐₜ*+1);
- ***P*_{*k*+1*,k*}** : matrice de covariance de l'erreur à l'instant *k +* 1 prédite à l'instant *k* : dimension (*Nₑₜₐₜ*+1, *Nₑₜₐₜ*+1) *;*
- ***Kₖ*** : matrice de gain de Kalman calculée à l'instant *k* : dimension (*Nₑₜₐₜ*+1, *M)*
- ***yₖ*** : mesure observée à l'instant k : dimension (M). Dans cet exemple, la mesure est un scalaire : M = 1.
- ***H̃*** : matrice d'observation : dimension (*Nₑₜₐₜ*+1,M) *;*
- *G* : matrice de commande : dimension (*Nₑₜₐₜ*+1, Q) ;
- ***w̃ₖ*** : bruit de modélisation : dimension (*Nₑₜₐₜ*+1) *;*
- ***vₖ*** : bruit d'observation : dimension (M) ; Il est supposé que les bruits d'observation et de modélisation sont blancs et indépendants ;
- ***Rₖ*** : matrice de covariance du bruit d'observation à l'instant k. Dimension (M, M) ;
- ***Qₖ*** : matrice de covariance du bruit du système à l'instant k. Dimension (*Nₑₜₐₜ*+1, *Nₑₜₐₜ*+1) *;*
- ***F̃*** : matrice de transition augmentée de dimensions (*Nₑₜₐₜ + 1, Nₑₜₐₜ +* 1). $w \left[k\right] ∼ N \left(0, {σ}_{w}^{2}\right)$ est le bruit qui intègre les erreurs liées au modèle et $w \left[k\right] ∼ N \left(0, {σ}_{v}^{2}\right)$ décrit le bruit sur les valeurs fournies par le capteur. Leurs matrices de covariances sont définies : $E \left[{w}_{k}{w}_{n}^{T}\right] = \left\{\begin{matrix}{Q}_{k} , k \neq n \\ 0 , k = n\end{matrix}\right.$ $E \left[{v}_{k}{v}_{n}^{T}\right] = \left\{\begin{matrix}{R}_{k} , k \neq n \\ 0 , k = n\end{matrix}\right.$
$N \left(0, {σ}_{w}^{2}\right)$ signifie une loi normale de moyenne nulle et de variance ${σ}_{w}^{2}$.
~ signifie est distribué selon
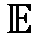 désigne l'opérateur espérance
*w[k]* et v[k] sont supposés et mutuellement indépendants. $E \left[{w}_{k}{v}_{n}^{T}\right] = 0$

Le procédé comporte une phase d'initialisation, au cours de laquelle on utilise un vecteur d'état initial ***c*_{*k*=1*,*0}** et une matrice de covariance de l'erreur à l'instant initial ***P*_{*k*=1,0}**.

L'étape de correction est définie par les équations suivantes :
Le gain de Kalman est estimé par ${K}_{k} = {P}_{k , k - 1} {{H}^{˜}}^{T} {\left({H}^{˜}{P}_{k , k - 1}{{H}^{˜}}^{T}+{R}_{k}\right)}^{- 1}$

Le gain de Kalman traduit les poids (i-e la confiance) donnés à la mesure bruitée ***yₖ*** et à son estimée ***H̃c̃*_{*k,k*-1}**

Estimation de la concentration ***c̃_{k,k}*** à l'instant k : ${{c}^{˜}}_{k , k} = {{c}^{˜}}_{k , k - 1} + {K}_{k} \left({y}_{k}-{H}^{˜}{x}_{k , k - 1}\right)$

Estimation de la matrice de covariance de l'erreur d'estimation ***P_{k,k}*** : ${P}_{k , k} = \left(I-{K}_{k}{H}^{˜}\right) {P}_{k , k - 1} {\left(I-{K}_{k}{H}^{˜}\right)}^{T} + {K}_{k} {R}_{k} {K}_{k}^{T}$

L'estimation de ***c̃_{k,k}*** permet de connaître *C_{in blood}*, ce dernier étant le terme de rang 1 du vecteur ***c̃_{k,k}***. A partir de *C_{in blood}*, on peut estimer ${P}_{{CO}_{2}}^{in blood}$*.*

L'étape de prédiction est définie par les équations suivantes :
Prédiction de la concentration à l'instant k+1 : ${A}^{˜} {{c}^{˜}}_{k + 1 , k} = {{c}^{˜}}_{k , k} + {G}^{˜} {C}_{k + 1}^{in col air}$

Prédiction de la matrice de covariance de l'erreur ***P*_{*k*+1*,k*}** ${P}_{k + 1 , k} = {{A}^{˜}}^{- 1} {P}_{k , k} {{A}^{˜}}^{- {1}^{T}} + {Q}^{˜}$

La variable que l'on cherche à estimer *C_{in blood}*, se trouve dans le vecteur d'état ***c̃****ₖ* à la position *z*_{*deb*1} (terme de rang 1) Elle est liée à la variable d'état suivante *C*_{*blood*1} par les termes de l'équation de transport. L'équation d'état qui lui est associée est : ${C}_{in blood} \left[k+1\right] = {φC}_{in blood} \left[k\right] + w \left[k+1\right]$

Où *φ* est un paramètre caractérisant notre a priori sur le signal *C_{in blood}* . Si notre a priori est que le signal en entrée est stationnaire, nous choisirons *φ =* 1

L'expression (50) se traduit par : ${A}^{˜} {{c}^{˜}}_{k + 1} = {{c}^{˜}}_{k} + {G}^{˜} {q}_{k + 1} + {{w}^{˜}}_{k + 1}$ où
- ***c̃****ₖ* est le vecteur d'état augmenté précédemment défini. ***c̃****ₖ* = ***c̃***(*tₖ*). Le vecteur d'état augmenté comporte la concentration dans chaque compartiment ainsi que la concentration dans le sang. On définit pour un schéma d'intégration temporel implicite ${A}^{˜} = I - {F}^{˜} ⋅ δt$

Où *la matrice de transition augmentée* ***F̃*** *est*
- ***F̃***_{[(*N*+1)*x*(*N*+1)]} est la matrice définie par: ${F}^{˜} = \left[\begin{matrix}φ & 0 \\ {g}_{11} & \\ 0 & \\ 0 & \left[F\right] \\ 0 & \\ 0 & \end{matrix}\right]$ ${G}^{˜} = \left[\begin{matrix}0 \\ ⋯ \\ 0 \\ g \\ 0 \\ ⋯ \\ 0\end{matrix}\right]$

Où $g = 2 \frac{{u}_{x}^{col}}{Δ x}$ pour le terme défini par la hauteur suivant l'axe z : *z*_{*deb* 4} + *δz*₄
et
***w̃*** est le vecteur contenant le bruit sur les états. Il a la dimension (*Nₑₜₐₜ + 1)x1* ${w}^{˜} = \left(\begin{matrix}{w}^{in blood} \\ ⋯ \\ w\end{matrix}\right)$

Pour un schéma implicite d'intégration dans le temps, l'équation d'état discrète s'écrit : ${A}^{˜} {c}^{˜} \left[k+1\right] = {c}^{˜} \left[k\right] + {G}^{˜} {C}^{in col air} \left[k+1\right] + {w}^{˜} \left[k+1\right]$

*w^{in blood}* suit une loi normale $N \left(0, {σ}_{w}^{2}\right)$
et ${q}_{k + 1} = \left({C}_{{CO}_{2}}^{in col air}\left({z}^{col 1},k+1\right)\right)$

Où la matrice ***Ã*** de dimension (*Nₑₜₐₜ +* 1)*x*(*Nₑₜₐₜ* + 1) est définie par : ${A}^{˜} = I - {F}^{˜} ⋅ δt$

Où ***I*** est la matrice identité de dimension (*Nₑₜₐₜ +* 1)*x*(*Nₑₜₐₜ* + 1)

L'équation d'observation pour le système augmenté s'écrit : ${y}_{k} = {{h}^{˜}}^{t} {{c}^{˜}}_{k} + {v}_{k}$

Où :
- *yₖ* est l'observation bruitée à partir de la valeur réelle de concentration ***c̃****ₖ.* ${y}_{k} = {C}_{{CO}_{2}}^{mes 3}$
- $v \left[k\right] ∼ N \left(0, {σ}_{v}^{2}\right)$ décrit le bruit d'observation sur les valeurs fournies par le capteur
- ***h̃*** est le vecteur d'observation augmenté défini par : ${h}^{˜} = \left[\begin{matrix}0 \\ h\end{matrix}\right]$

Dans notre cas, comme l'observation est définie par une seule valeur, la matrice d'observation augmentée ***H̃*** se réduit à un vecteur d'observation ***h̃****.* ${y}_{k} = {{H}^{˜}}^{t} {{c}^{˜}}_{k} + {v}_{k}$

Où : ${H}^{˜} = {h}^{˜}$

Le système d'équations est $\left\{\begin{matrix}{A}^{˜} {{c}^{˜}}_{k + 1} = {{c}^{˜}}_{k} + {G}^{˜} {C}_{k + 1}^{in col air} + {{w}^{˜}}_{k + 1} \\ {y}_{k} = {{H}^{˜}}^{t} {{c}^{˜}}_{k} + {v}_{k}\end{matrix}\right.$

L'algorithme de Kalman enchaine de façon récursive une étape de correction du vecteur d'état à un instant *k* (ou *tₖ*) et de prédiction du vecteur d'état
A partir de *C_{in blood}*, la teneur du sang en CO2 peut être ${P}_{{CO}_{2}}^{in blood} = \frac{{C}_{in blood}}{{β}^{sang}}$
- *β^{sang}* est le coefficient de solubilité d'Ostwald du gaz carbonique dans le sang.

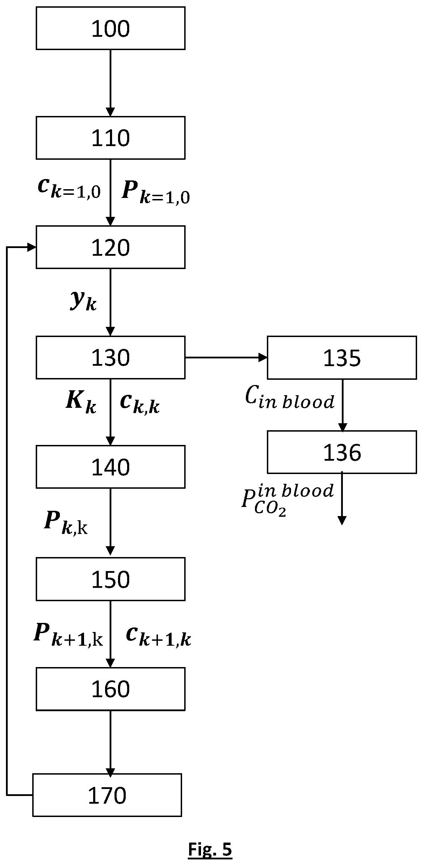

La figure 5 schématise les principales étapes de mise en œuvre d'un procédé selon l'invention.

Etape 100 : apposition du dispositif de mesure contre le milieu analysé et activation de la pompe.

Etape 110 : initialisation : Au cours de cette étape, on définit un vecteur d'état initial ***c***_{***k**=***1**,**0**} et une matrice de covariance de l'erreur à l'instant initial ***P*_{*k*=1,0}**. L'instant de mesure k est ensuite incrémenté : k = 2.

Etape 120 : mesure de la concentration de CO₂ dans la chambre de mesure. On obtient ainsi la grandeur mesurée ***yₖ**.*

Etape 130 : A partir du vecteur d'état initial ***c***_{***k**=***1,0**}*,* ou du vecteur d'état estimé lors d'une itération précédente ***c*_{*k*,*k*-1}**,et de la matrice de covariance de l'erreur à l'instant initial ***P*_{*k*=1,0}**, ou résultant d'une itération précédente ***P*_{*k*,*k*-1}**, détermination du gain de Kalman à l'instant k (expression 60) et estimation du vecteur d'état ***c_{k,k}*** (expression 61).

L'estimation de ***c_{k,k}*** permet de connaître *C_{in blood}*, ce dernier étant le terme de rang *z*_{*deb*1} du vecteur ***c_{k,k}*** : cela correspond à l'étape 135. A partir de *C_{in blood}*, on peut estimer ${P}_{{CO}_{2}}^{in blood}$ (expression 67) : étape 136.

Etape 140 : Estimation de la matrice de covariance ***P_{k,k}*** de l'erreur d'estimation (expression 62).

Etape 150 : Prédiction du vecteur d'état ***c*_{*k,k*+1}** à l'instant k+1.(expression 62') et prédiction de la matrice de covariance de l'erreur à l'instant suivant ***P*_{*k*+1*,k*}**.

Etape 160 : incrémentation de l'instant de mesure

Etape 170 : réitération des étapes 110 à 160 ou sortie de l'algorithme

Les étapes itératives 110 à 170 sont réitérées jusqu'à la sortie de l'algorithme. La sortie de l'algorithme peut être décidée par l'utilisateur, ou correspondre à un nombre prédéterminé d'itérations.

On a mis en œuvre le procédé précédemment décrit en prenant en compte les paramètres suivants :
- longueur (selon l'axe X) compartiment sang: Δ*x^{sang}* = Δ*x* =5 cm ;
- largeur (selon l'axe Y) compartiment sang : Δ*y^{sang}* = Δ*y* =2 cm ;
- hauteur compartiment sang (selon l'axe Z) : Δ*z^{sang}* = 0.3 cm ;
- surface du compartiment sang au contact de la peau : *A^{sang}* = 10 cm² ;
- volume du compartiment sang : *V^{sang}* = 3 cm³ ;
- Coefficient de Henry du gaz carbonique dans le sang : *H^{sang}* = 0,54
- Coefficient de diffusion du gaz carbonique dans le sang (coefficient de l'eau) : *D^{sang}* = 2,2 10⁻⁵ cm².s⁻¹
- Concentration initiale en CO₂ dans le sang : *C^{in sang}* = 1.0990 µmol/cm3
- Débit sanguin : *Q^{sang}* = 1.83 10⁻³ ml/s (cm³s⁻¹) qui correspond à la vitesse suivante:
   o Vitesse transverse (selon x) : ${u}_{x}^{sang} = 0 , 0031 cm {. s}^{- 1}$
   o Vitesse axiale (selon z) : ${u}_{z}^{sang} = 1.83 {10}^{- 4} cm {. s}^{- 1}$
- longueur compartiment peau (selon l'axe X) *:* Δ*x^{peau}* = Δ*x* =5 cm ;
- largeur compartiment peau (selon l'axe Y): Δ*y^{peau}* = Δ*y* =2 cm ;
- hauteur compartiment peau (selon l'axe Z): Δ*z^{peau}* =16 10⁻⁴ cm ;
- surface de la peau en contact avec le dispositif : *A^{peau}* =10 cm² ;
- Coefficient de Henry du gaz carbonique dans la peau : *H^{peau}* =1,6
- Coefficient de diffusion du gaz carbonique dans la peau : *: D^{peau}* = 1 10⁻⁷ cm².s⁻¹
- température de la chambre de mesure : *T^{mes}* = 315.15 K ;
- longueur de la chambre de mesure (selon l'axe X): Δ*x^{mes}* = Δ*x* =5 cm ;
- largeur de la chambre de mesure (selon l'axe Y): Δ*y^{mes}* = Δ*y* =2 cm ;
- hauteur de la chambre de mesure (selon l'axe Z): Δ*z^{mes}* = 0.25 cm ;
- Coefficient de Henry du gaz carbonique dans la chambre de mesure : *H^{mes}* = *H^{air}* =1
- Coefficient de diffusion du gaz carbonique dans la chambre de mesure : *D^{mes}* = *D^{air}* = 0,18 cm².s⁻¹
- Concentration en Co₂ dans l'air entrant, on suppose qu'il y a un filtre : *C^{air}*=0
- Débit d'air convectif dans la chambre de collecte : *Q^{air}* = 1.67 10⁻² ml/s
   o Vitesse transversale (selon l'axe x) : ${u}_{x}^{col} = 33 {10}^{- 3} cm {. s}^{- 1}$
   o Vitesse axiale (selon l'axe z) : ${u}_{z}^{col} = {1,67 . 10}^{- 3} cm {. s}^{- 1}$
- surface de la face de contact entre la chambre de mesure et la chambre de collecte : *A^{mes}* = *A^{col}* =10 cm² ;
- température de la chambre de collecte : *T^{col}* = 315.15 K ;
- longueur de la chambre de collecte (selon l'axe X) *:* Δ*x^{col}* = Δ*x* =5 cm ;
- largeur de la chambre de collecte (selon l'axe Y): Δ*y^{col}* = Δ*y* =2 cm ;
- hauteur de la chambre de collecte (selon l'axe Z): Δ*z^{col}* = 0.25 cm ;
- Coefficient de Henry du gaz carbonique dans la chambre de collecte : *H^{col} = H^{air}* =1
- Coefficient de diffusion du gaz carbonique dans la chambre de collecte : *D^{col} = D^{air}* = 0,18 cm².s⁻¹
- Facteur de transparence entre peau et la cellule de mesure : r_{peau|mes}=1 ;
- Facteur de transparence entre la cellule de mesure et la cellule de collecte : r_{mes|col}=1
- Pas d'échantillonnage temporel : *δt* = 1s
- Variance du bruit de mesure : 10⁻⁶ mol/m³
- Variance du bruit du modèle : 10⁻⁸ mol/m³
- Coefficient du modèle autorégressif : *φ =* 0

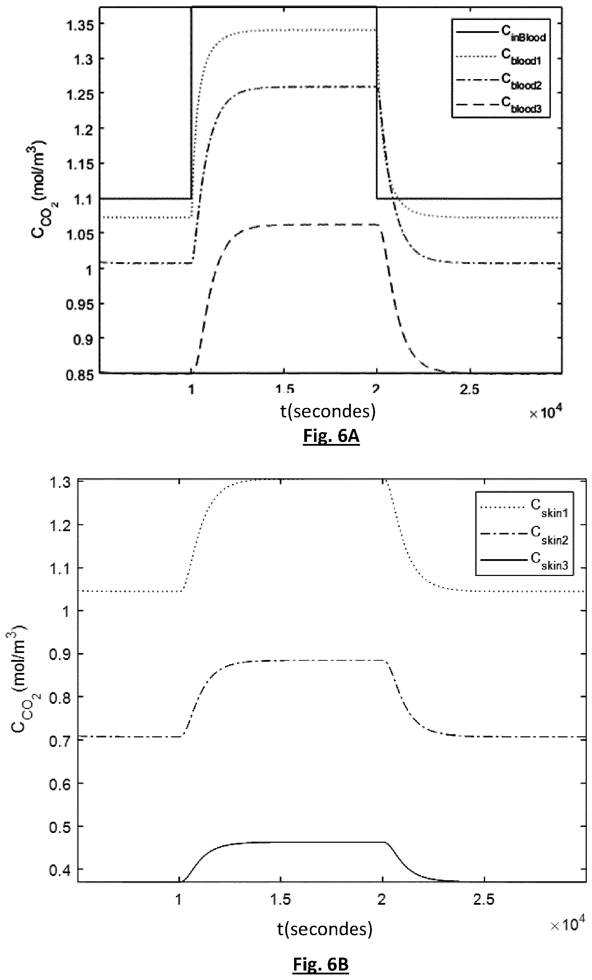

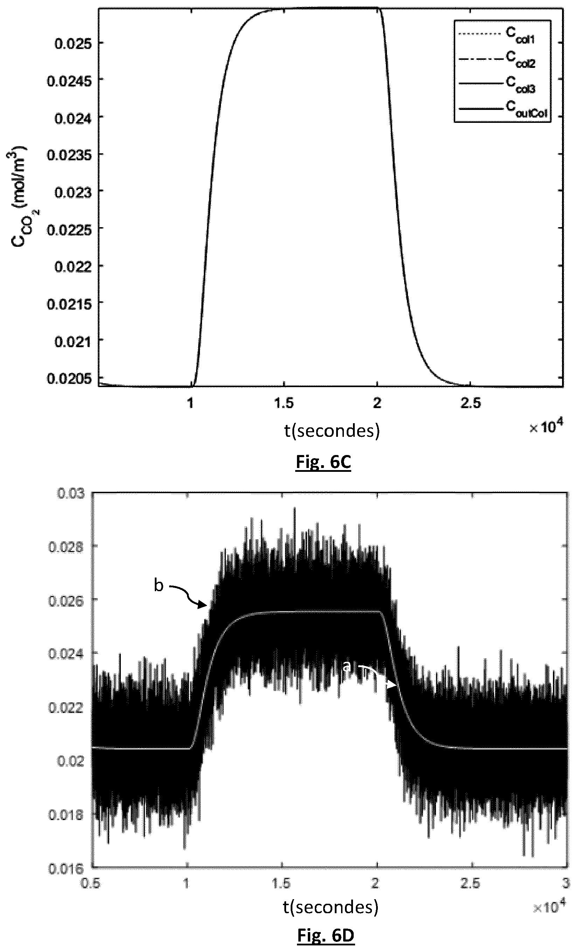

On a tout d'abord mis en œuvre un modèle direct, permettant d'estimer la concentration en chaque point d'échantillonnage à partir d'une concentration connue dans le sang. La concentration connue dans le sang suit un créneau. Les figures 6A, 6B, 6C décrivent l'évolution de la concentration en fonction du temps respectivement dans le compartiment sang, dans la peau, et dans la chambre de collecte. La figure 6D montre l'évolution de la concentration au niveau du capteur de gaz, dans la chambre de mesure (courbe a). La courbe b de la figure 6D montre la concentration au niveau du capteur de gaz de la courbe a, à laquelle on a ajouté un bruit gaussien pour simuler des mesures bruitées. Sur chacune des figures 6A à 6D, l'axe des abscisses correspond au temps et l'axe des ordonnées correspond à la concentration de CO2 estimée.

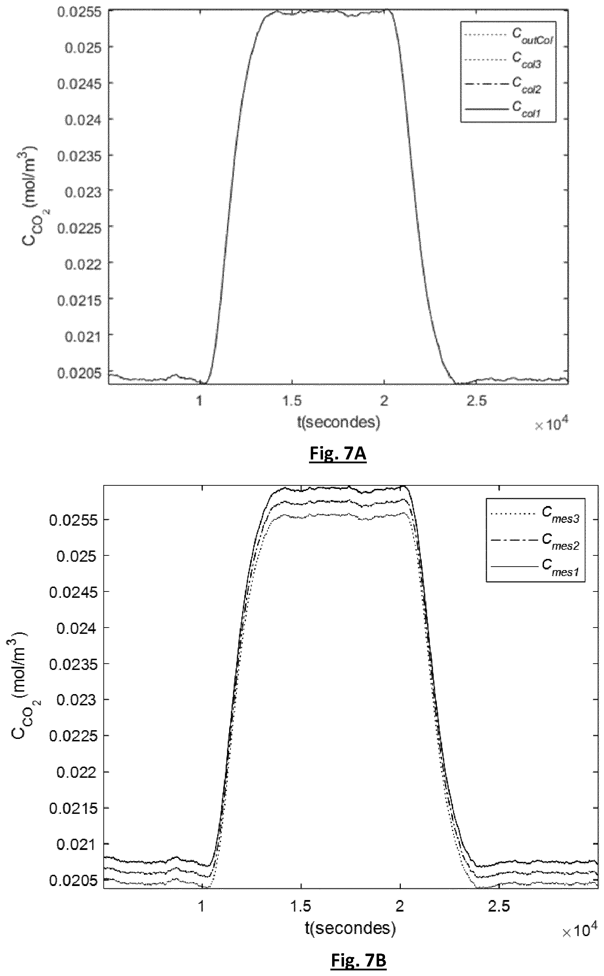

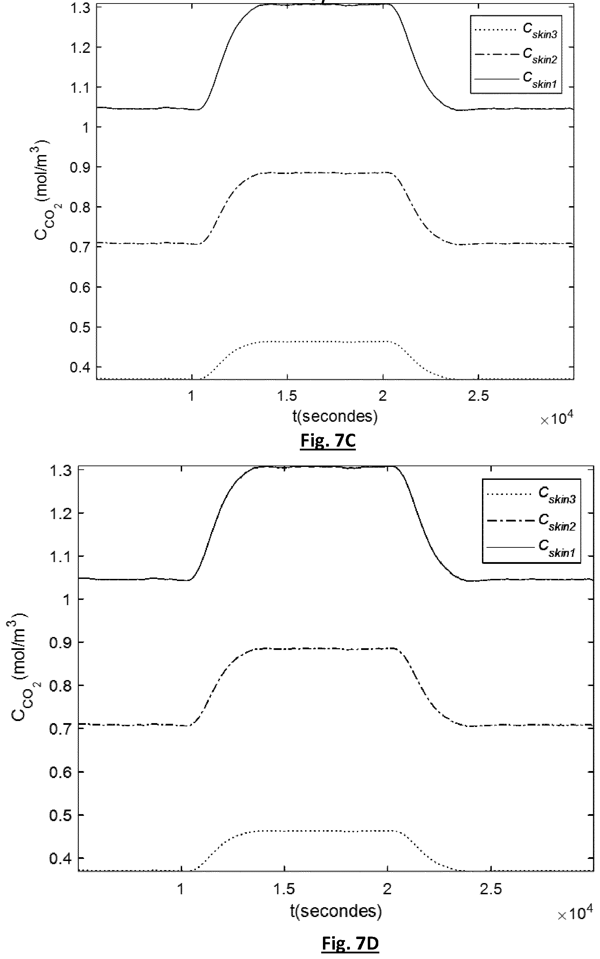

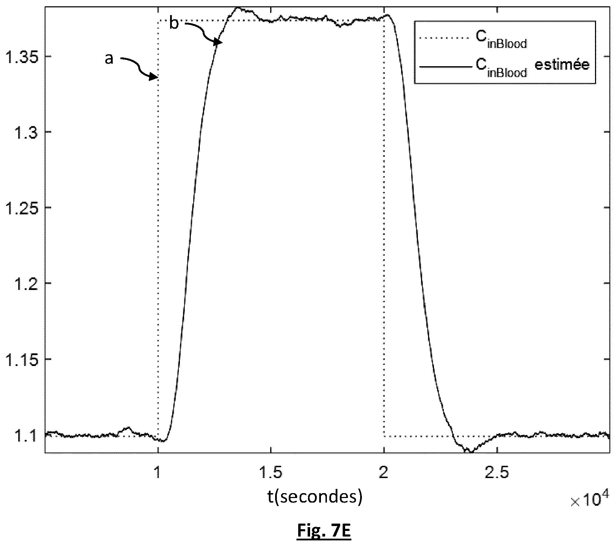

A partir des mesures bruitées simulées (courbe b de la figure 6D), on a mis en œuvre l'estimateur récursif précédemment décrit. Le vecteur d'état estimé à chaque instant de mesure permet d'obtenir une estimation de la concentration en différents points d'échantillonnage. Les figures 7A, 7B, 7C, 7D montrent l'évolution de la concentration de CO2 respectivement au niveau de la chambre de collecte, de la chambre de mesure, du compartiment peau et du sang. La figure 7E montre la concentration dans le sang réelle (courbe a) et la concentration dans le sang résultant de l'estimation.

Sur chacune des figures 7A à 7E, l'axe des abscisses correspond au temps et l'axe des ordonnées correspond à la concentration de CO2 estimée.

### Deuxième modèle : modèle bidimensionnel 2D

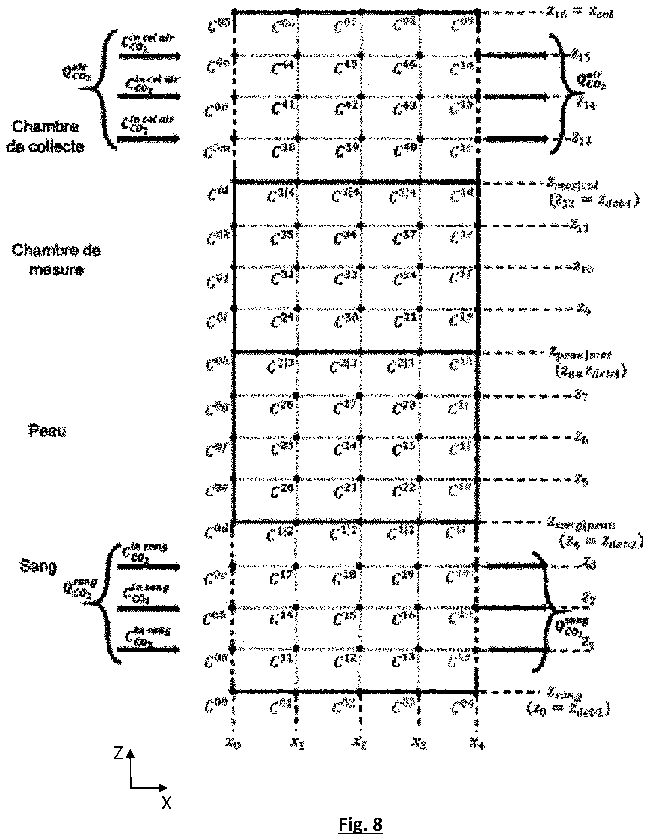

Nous décrivons dans la suite un modèle selon lequel l'évacuation dans la cellule de collecte se fait de façon transverse (perpendiculairement à l'axe z), comme représenté sur la figure 8. La géométrie choisie fixe le calcul du champ de vecteur vitesse pour la propagation de l'air porteur dans la cellule de collecte, les équations de convection diffusion définissent la propagation du gaz carbonique. L'entrée d'air se fait par une ou les deux faces latérales.

La propagation du sang est modélisée par une circulation selon un axe X, perpendiculaire à l'axe Z.

Chacun des compartiments définis précédemment est discrétisé aussi selon l'axe x, de la même manière que selon z, à savoir avec 3 valeurs de concentration internes aux compartiments et deux valeurs de concentrations sur les bords.

La figure 8 représente le découpage en compartiments. Sur cet exemple, l'indice de position en X varie de 0 à 4 comme indiqué en bas de la figure. L'indice de position selon l'axe z varie de 0 à 16 avec les bords et de 1 à 15 pour les points internes, comme indiqué à droite de la figure. On utilise la notation d'indice *kₓ ou k_{z}* selon l'axe étudié. ${k}_{x} ∈ \left\{0, 1, 2, 3, 4\right\}$ ${k}_{z} ∈ \left\{0, 1, 2, 3, 4, 5, 6, 7, 8, 9, 10, 11, 12, 13, 14, 15, 16\right\}$

Par exemple pour le compartiment « Peau », selon l'axe X l'indice *kₓ* varie de 0 à 4 pour les points comprenant les bords et de 1 à 3 pour les points internes ; selon l'axe Z l'indice *k_{z}*varie de 4 à 8, avec les interfaces et de 5 à 7 pour les points internes.

Au sein d'un milieu homogène, l'équation (11) devient : $\frac{\partial C \left(x, z, t\right)}{\partial t} = - {u}_{x} \frac{\partial C \left(x, z, t\right)}{\partial x} + D \left(\frac{{\partial }^{2} C \left(x, z, t\right)}{\partial {z}^{2}}+\frac{{\partial }^{2} C \left(x, z, t\right)}{\partial {x}^{2}}\right)$

Les conditions aux limites sont :
Au bord inférieur - niveau *z*₀, l'équation (13) devient $\frac{\partial C \left({x}_{{k}_{x}}, {z}_{0}, t\right)}{\partial z} = 0 ; {k}_{x} ∈ \left\{0, 1, 2, 3, 4\right\}$

Au **bord supérieur** - niveau *z_{col}* = *z*₁₆, l'équation (13) devient $\frac{\partial C \left({x}_{{k}_{x}}, {z}_{16}, t\right)}{\partial z} = 0 ; {k}_{x} ∈ \left\{0, 1, 2, 3, 4\right\}$

Le long du côté gauche, à la coordonnée *x*₀ :
- Au bord latéral gauche de la cellule de sang $\frac{\partial C \left({x}_{0}, {z}_{{k}_{z}}, t\right)}{\partial x} = 0 ; {k}_{x} ∈ \left\{0\right\}$ $C \left({x}_{0}, {z}_{{k}_{z}}, t\right) = {C}_{in sang} \left(t\right) ; {k}_{z} ∈ \left\{1, 2, 3\right\}$
- Le long du côté gauche du compartiment peau et de la chambre de mesure : $\frac{\partial C \left({x}_{0}, {z}_{{k}_{z}}, t\right)}{\partial x} = 0 ; {k}_{z} ∈ \left\{4,…,12\right\}$
- Au bord latéral gauche de la cellule de collecte $\frac{\partial C \left({x}_{0}, {z}_{{k}_{z}}, t\right)}{\partial x} = 0 ; {k}_{z} ∈ \left\{16\right\}$ $C \left({x}_{0}, {z}_{{k}_{z}}, t\right) = {C}_{in col air} \left(t\right) ; {k}_{z} ∈ \left\{13, 14, 15\right\}$
- Le long du côté droit, selon la coordonnée *x*₄ : $\frac{\partial C \left({x}_{4}, {z}_{{k}_{z}}, t\right)}{\partial x} = 0 ; {k}_{z} ∈ \left\{0,1,…,16\right\}$

### Conditions aux interfaces

Notation : l'indice *i* sera utilisé comme référence au milieu avant l'interface, l'indice *j* pour le milieu après l'interface. La coordonnée à l'interface est indiquée par l'indice *i*|*j.*

Continuité du flux, l'équation (18) devient ${r}_{transpa i \left|j\right.} {D}_{i} \frac{\partial {C}_{i} \left(x, {z}_{i \left|j\right.}, t\right)}{\partial z} = {D}_{j} \frac{\partial {C}_{j} \left(x, {z}_{i \left|j\right.}, t\right)}{\partial z}$

Continuité de la pression l'équation (21) devient $\frac{{C}_{i} \left(x, {z}_{i \left|j\right.}, t\right)}{{H}_{i}} = \frac{{C}_{j} \left(x, {z}_{i \left|j\right.}, t\right)}{{H}_{j}} {=}^{def} {C}_{i \left|j\right.} \left(x, {z}_{i \left|j\right.}, t\right)$

### Expression de la vitesse de l'air.

Les notations utilisées sont :
- *ρₐᵢᵣ* la masse volumique de l'air
- *Mₐᵢᵣ* la masse molaire de l'air dans les proportions standard de ses différents composants
- *Cₐᵢᵣ* la concentration molaire volumique de l'air dans les proportions standard de ses différents composants
- *Pₐᵢᵣ* la pression de l'air
- *uₐᵢᵣ* la vitesse de l'air
- *γₐᵢᵣ* l'accélération de l'air
- *Tₐᵢᵣ* la température de l'air
- *Fⱼ* champ de force massique traduisant l'effet de la gravité sur le gaz.

Nous avons la relation : ${ρ}_{air} = {C}_{air} . {M}_{air}$

D'après la loi des gaz parfaits nous avons : ${P}_{air} = \frac{{ρ}_{air}}{{M}_{air}} R . {T}_{air}$

L'équation de conservation de la quantité de mouvement conduit à l'équation de Navier Stokes. Comme la vitesse est faible, nous pouvons faire l'hypothèse que le gaz est incompressible et que la masse volumique reste constante. Nous négligeons aussi l'effet de la gravité sur le gaz et la viscosité de l'air. L'équation simplifiée devient : ${ρ}_{air} {γ}_{air} = - grad \left({P}_{air}\right)$

Où : ${γ}_{air} = \frac{{du}_{air}}{dt} + {u}_{air} . \overline{\overline{grad}} \left({u}_{air}\right)$

Où $\overline{\overline{grad}} \left({u}_{air}\right)$ est le tenseur : $\overline{\overline{grad}} \left({u}_{air}\right) = \left[\frac{\partial {u}_{air}}{\partial x}\right]$

L'équation de conservation de la masse s'écrit: $\frac{\partial {ρ}_{air}}{\partial t} + div \left({ρ}_{air}{u}_{air}\right) = 0$

Soit en régime stationnaire : $div \left({ρ}_{air}{u}_{air}\right) = 0$

Si nous supposons que *ρₐᵢᵣ* est constant dans la chambre du dispositif, nous obtenons comme équation simplifiée : $div \left({u}_{air}\right) = 0$

La connaissance du débit d'air en entrée de la cellule de collecte fixe la valeur de *uₐᵢᵣ* en entrée du dispositif. La résolution de cette équation permet de calculer le champ de vitesse à l'intérieur du dispositif.

Dans le cas d'une propagation suivant une seule direction, par exemple X, l'équation devient : $\frac{{du}_{x , air}}{dx} = 0$

Le champ de vitesse *u_{x,air}* est constant dans tout le dispositif et égal au vecteur vitesse en entrée du dispositif.

Nous notons (*x*₁,*x*₂) les coordonnées spatiales (*x*, *z*) et (*u*_{1,*air*},*u*_{2,*air*}) les coordonnées du vecteur vitesse de **l'air** *uₐᵢᵣ* = (*u*_{*x*,*air*},*u*_{*z*,*air*}): $div \left({u}_{air}\right) = 0$

Soit encore : ${\sum }_{i = 1}^{2} \frac{\partial {u}_{i , air}}{\partial {x}_{i}} = 0$

Et : $\frac{\partial {u}_{j , air}}{\partial t} + {\sum }_{i = 1}^{2} {u}_{i , air} \frac{\partial {u}_{j , air}}{\partial {x}_{i}} = \frac{1}{{ρ}_{air}} \left[-\frac{\partial {P}_{air}}{\partial {x}_{j}}+{F}_{j}\right] j = 1 , 2$

A l'état stationnaire, en supposant négligeable l'effet de la gravité sur le gaz (*Fⱼ* = 0), cette équation devient : ${\sum }_{i = 1}^{2} {u}_{i , air} \frac{\partial {u}_{j , air}}{\partial {x}_{i}} = - \frac{1}{{ρ}_{air}} \frac{\partial {P}_{air}}{\partial {x}_{j}} j = 1 , 2$

Equations de Navier Stokes pour un fluide parfait visqueux incompressible en 2D : Nous notons (*x*₁,*x*₂) les coordonnées spatiales (*x, z*) et (*u*₁*_{,air},u_{2,air}*) les coordonnées du vecteur vitesse (*u_{x,air},u_{z,air}*) : $div \left({u}_{air}\right) = 0$

Soit encore : ${\sum }_{i = 1}^{2} \frac{\partial {u}_{i , air}}{\partial {x}_{i}} = 0$

Et : $\frac{\partial {u}_{j , air}}{\partial t} + {\sum }_{i = 1}^{2} {u}_{i , air} \frac{\partial {u}_{j , air}}{\partial {x}_{i}} = \frac{1}{{ρ}_{air}} \left[-\frac{\partial {P}_{air}}{\partial {x}_{j}}+{F}_{j}+ν{\sum }_{i = 1}^{2} \frac{{\partial }^{2} {u}_{j , air}}{\partial {x}_{i}^{2}}\right] j = 1 , 2$

Où v est la viscosité cinétique du fluide (unité : *m*²*s*⁻¹).

A l'état stationnaire, en supposant négligeable l'effet de la gravité sur le gaz (*Fⱼ* = 0), cette équation devient : ${\sum }_{i = 1}^{2} {u}_{i , air} \frac{\partial {u}_{j , air}}{\partial {x}_{i}} = \frac{1}{{ρ}_{air}} \left[-\frac{\partial {P}_{air}}{\partial {x}_{j}}+ν{\sum }_{i = 1}^{2} \frac{{\partial }^{2} {u}_{j , air}}{\partial {x}_{i}^{2}}\right] j = 1 , 2$

Dans le cas du modèle 2D avec évacuation transverse, nous proposons d'utiliser un modèle simplifié du champ de vitesse en supposant qu'il est uniforme dans toute la chambre, et parallèle à l'axe x. L'entrée d'air se fait par un seul côté et l'équation (25) devient: ${u}_{x}^{col} = \frac{{Q}^{air}}{{A}_{x}^{in col}}$

Où *Q^{air}* est le débit d'air et ${A}_{x}^{in col}$ est la surface d'entrée le long de la paroi latérale de la cellule 5 de collecte, ${A}_{x}^{out col}$ est la surface de sortie le long de la paroi latérale de la cellule 5 de collecte. Nous supposons de plus que ${A}_{x}^{out col} = {A}_{x}^{in col}$.

Nous supposons que la vitesse axiale est nulle. ${u}_{z}^{col} = 0$

### Expression de la vitesse du sang.

${A}_{x}^{in sang}$ est la surface d'entrée à gauche dans le compartiment sang. ${A}_{x}^{out sang}$ est la surface de sortie à droite du compartiment sang. Il s'agit de modéliser une section des vaisseaux sanguins. Nous supposons de plus que ${A}_{x}^{out sang} = {A}_{x}^{in sang}$*.* Nous supposons que l'entrée se fait par tous les sous-compartiments du compartiment sang, c'est-à-dire pour *k_{z}* ∈ {1,2,3}. ${u}_{x}^{sang} = \frac{{Q}^{sang}}{{A}_{x}^{out sang}}$

Nous supposons que la vitesse axiale est nulle : ${u}_{z}^{sang} = 0$

La vitesse de convection dans la cellule de mesure est supposée nulle: ${u}_{x}^{mes} = 0$ ${u}_{z}^{mes} = 0$

La vitesse de convection dans la peau est supposée nulle: ${u}_{x}^{peau} = 0$ ${u}_{z}^{peau} = 0$

En résumé, sur tout le modèle 2D, le champ de vitesse de convection discrétisé sur les points d'échantillonnage s'écrit: ${u}_{x} \left(x, {z}_{{k}_{z}}, t\right) = \left\{\begin{matrix}0 & ; {k}_{z} = 0 \\ {u}_{x}^{sang} & ; {k}_{z} ∈ \left\{1, 2, 3\right\} \\ 0 & ; {k}_{z} ∈ \left\{4,…,12\right\} \\ {u}_{x}^{col} & ; {k}_{z} ∈ \left\{13, 14, 15\right\} \\ 0 & ; {k}_{z} = 16\end{matrix}\right.$ ${u}_{z} \left(x, {z}_{{k}_{z}}, t\right) = \left\{\begin{matrix}0 & ; {k}_{z} = 0 \\ 0 & ; {k}_{z} ∈ \left\{1, 2, 3\right\} \\ 0 & ; {k}_{z} ∈ \left\{4,…,12\right\} \\ 0 & ; {k}_{z} ∈ \left\{13, 14, 15\right\} \\ 0 & ; {k}_{z} = 16\end{matrix}\right.$

### Discrétisation du modèle 2D

Pour la discrétisation du modèle 2D continu, la démarche reste la même que pour le modèle 1D précédemment décrit. A partir des équations continues nous établissons les équations discrètes en utilisant la méthode des différences finies centrées. Nous formulons le problème sous une forme vectorielle/matricielle afin d'employer le filtre de Kalman pour l'estimation des état cachés du système, et pour l'estimation de la variable d'intérêt *C_{in sang}*.

Le maillage spatial est réalisé selon les deux axes X et Z comme représenté sur la figure 8.

De même qu'en 1D, nous définissons la distance *δzᵢ* entre deux points successifs pour chaque milieu *i* selon z: ${δz}_{i} = \frac{Δ {z}_{i}}{{N}_{z , i} - 1}$

Où *N_{z,i}* est le nombre total de points d'échantillonnage suivant l'axe z pour le milieu i (*N_{z,i}* = 5 dans notre cas).

La position suivant z du début du milieu i dans la colonne est notée *z_{deb i}* . Elle est définie par l'expression ${z}_{deb i} = \left\{\begin{matrix}{z}_{0} & pour i = 1 \\ {z}_{0} + \left({\sum }_{j = 1}^{i - 1} Δ {z}_{j}\right) & pour i ∈ \left\{2, 3, 4\right\}\end{matrix}\right.$

Où *z*₀ est la cote suivant l'axe Z du bord inférieur du compartiment sang et Δ*zⱼ* est la hauteur du milieu j.

Nous procédons de la même manière selon l'axe X :
Nous connaissons la largeur supposée égale de chaque milieu : Δ*xᵢ* = Δ*x*
Nous choisissons le nombre de points de discrétisation supposé le même pour chaque milieu selon l'axe X, dont (*Nₓ* - 2) points internes et deux points sur les bords, soit *Nₓ* points au total (*Nₓ* =5 dans notre cas). La distance *δx* entre deux points successifs pour chaque milieu *i* selon x est: $δx = \frac{Δ x}{{N}_{x} - 1}$

Les concentrations situées dans le vecteur ***c*₀** sont associées aux points d'échantillonnage spatial correspondant aux différentes hauteurs, puis aux différentes abscisses, et enfin aux différents milieux.

Les composantes du vecteur ***c*₀** sont ainsi définies de la manière suivante, en faisant varier d'abord l'indice *kₓ* correspondant à un balayage selon l'axe X en ajoutant les deux bords, puis en faisant varier l'indice *k_{z}* correspondant à un balayage selon l'axe Z en ajoutant les bords et les interfaces, puis en faisant varier l'indice *i* du milieu: ${c}_{0} = \left[\begin{matrix}C \left({x}_{0}+{k}_{x}.δx,{z}_{deb 1}+{k}_{z}.{δz}_{1}\right) ; & {k}_{x} = 0 , … , {N}_{x} - 1 ; {k}_{z} = 0 , … , \left({N}_{z , 1}-1\right) \\ C \left({x}_{0}+{k}_{x}.δx,{z}_{deb 2}+{k}_{z}.{δz}_{2}\right) ; & {k}_{x} = 0 , … , {N}_{x} - 1 ; {k}_{z} = 1 , … , \left({N}_{z , 2}-1\right) \\ C \left({x}_{0}+{k}_{x}.δx,{z}_{deb 3}+{k}_{z}.{δz}_{3}\right) ; & {k}_{x} = 0 , … , {N}_{x} - 1 ; {k}_{z} = 1 , … , \left({N}_{z , 3}-1\right) \\ C \left({x}_{0}+{k}_{x}.δx,{z}_{deb 4}+{k}_{z}.{δz}_{4}\right) ; & {k}_{x} = 0 , … , {N}_{x} - 1 ; {k}_{z} = 1 , … , \left({N}_{z , 4}-1\right)\end{matrix}\right]$ ${c}_{0} = \left[\begin{matrix}C \left({x}_{0}+0.δx,{z}_{deb 1}+0.{δz}_{1}\right) \\ … \\ C \left({x}_{0}+\left({N}_{x}-1\right).δx,{z}_{deb 1}+0.{δz}_{1}\right) \\ … \\ C \left({x}_{0}+0.δx,{z}_{deb 1}+\left({N}_{z , 1}-1\right).{δz}_{1}\right) \\ … \\ C \left({x}_{0}+\left({N}_{x}-1\right).δx,{z}_{deb 1}+\left({N}_{z , 1}-1\right).{δz}_{1}\right) \\ C \left({x}_{0}+0.δx,{z}_{deb 2}+1.{δz}_{2}\right) \\ … \\ … \\ C \left({x}_{0}+\left({N}_{x}-1\right).δx,{z}_{deb 2}+\left({N}_{z , 2}-1\right).{δz}_{2}\right) \\ C \left({x}_{0}+0.δx,{z}_{deb 3}+1.{δz}_{3}\right) \\ … \\ … \\ C \left({x}_{0}+\left({N}_{x}-1\right).δx,{z}_{deb 3}+\left({N}_{z , 3}-1\right).{δz}_{3}\right) \\ C \left({x}_{0}+0.δx,{z}_{deb 4}+1.{δz}_{4}\right) \\ … \\ … \\ C \left({x}_{0}+\left({N}_{x}-1\right).δx,{z}_{deb 4}+\left({N}_{z , 4}-1\right).{δz}_{4}\right)\end{matrix}\right]$

N définit le nombre total de points d'échantillons pris en compte pour la discrétisation spatiale du modèle de transport. Ces échantillons comprennent les points à l'intérieur strict des milieux, les points au bord supérieur et au bord inférieur, les points aux bords latéraux et les points qui correspondent aux trois interfaces sang/peau, peau/chambre de mesure, chambre de mesure/chambre de collecte $N = {N}_{x} . {N}_{z}$

Où *N_{z}* est le nombre total d'échantillons discrets en *z* , ${N}_{z} = 1 + {\sum }_{i = 1}^{4} \left[{N}_{z , i}-1\right]$

*Nₑₜₐₜ* regroupe les points aux bords (latéraux, supérieur et inférieur) et à l'intérieur strict des milieux en z en ne tenant pas compte des 3x(*Nₓ*) points qui correspondent aux interfaces sang/peau, peau/mes, mes/col.

Dans notre cas, nous avons : *Nₓ = 5; N*_{*z,*1} *= N*_{*z,*2} *= N*_{*z,*3} *= N*_{*z,*4} = 5 donc *N_{z}* = 17; *N* = 85 et *Nₑₜₐₜ* = 70.

Une fois défini ce vecteur ***c*₀**, la résolution du système suit la même procédure que celle décrite pour la résolution du système 1D.

Compte tenu de la discrétisation spatiale effectuée, l'équation de convection diffusion 2D-1 au sein d'un milieu homogène devient : $\begin{matrix}\frac{C \left(x,z,t+δt\right) - C \left(x, z, t\right)}{δt} \\ = \left[\frac{{u}_{x} δx + 2 D}{2 {\left(δx\right)}^{2}}\right] C \left(x-δx,z,τ\right) + \left[\frac{- 2 D}{{\left(δx\right)}^{2}}\right] C \left(x, z, τ\right) + \left[\frac{- {u}_{x} δx + 2 D}{2 {\left(δx\right)}^{2}}\right] C \left(x+δx,z,τ\right) \\ + \frac{2 D}{{δz}_{-} \left({δz}_{+}+{δz}_{-}\right)} C \left(x,z-{δz}_{-},τ\right) + \frac{- 2 D}{{δz}_{+} {δz}_{-}} C \left(x, z, τ\right) + \frac{2 D}{{δz}_{+} \left({δz}_{+}+{δz}_{-}\right)} C \left(x,z+{δz}_{+},τ\right)\end{matrix}$

*C(x, z, t)* est la concentration au point z qui suit la concentration *C*(*x, z - δz₋, t)* définie au point z - *δz*₋ et qui précède la concentration *C*(*x, z + δz*₊, *t*) définie au point z + *δz*₊ si l'on suit l'orientation (arbitraire) de l'axe z. *τ* indique le repère temporel.

Pour des pas d'échantillonnage égaux *δz*₊ = *δz₋* = *δzᵢ*, au sein d'un même milieu i homogène l'équation devient : $\begin{matrix}\frac{C \left(x,z,t+δt\right) - C \left(x, z, t\right)}{δt} \\ = \left[\frac{{u}_{x} δx + 2 {D}_{i}}{2 {\left(δx\right)}^{2}}\right] C \left(x-δx,z,τ\right) + \left[\frac{- 2 {D}_{i}}{{\left(δx\right)}^{2}}\right] C \left(x, z, τ\right) + \left[\frac{- {u}_{x} δx + 2 {D}_{i}}{2 {\left(δx\right)}^{2}}\right] C \left(x+δx,z,τ\right) \\ + \frac{2 {D}_{i}}{2 \left({δz}_{i}\right)} C \left(x,z-{δz}_{i},τ\right) - \frac{2 {D}_{i}}{{\left({δz}_{i}\right)}^{2}} C \left(x, z, τ\right) + \frac{2 {D}_{i}}{2 {\left({δz}_{i}\right)}^{2}} C \left(x,z+{δz}_{i},τ\right)\end{matrix}$

Soit encore : $\begin{matrix}\frac{C \left(x,z,t+δt\right) - C \left(x, z, t\right)}{δt} \\ = - 2 {D}_{i} \left[\frac{1}{{\left(δx\right)}^{2}}+\frac{1}{{\left({δz}_{i}\right)}^{2}}\right] C \left(x, z, τ\right) + \left[\frac{{u}_{x} δx + 2 {D}_{i}}{2 {\left(δx\right)}^{2}}\right] C \left(x-δx,z,τ\right) \\ + \left[\frac{- {u}_{x} δx + 2 {D}_{i}}{2 {\left(δx\right)}^{2}}\right] C \left(x+δx,z,τ\right) + \frac{2 {D}_{i}}{2 {\left({δz}_{i}\right)}^{2}} C \left(x,z-{δz}_{i},τ\right) + \frac{2 {D}_{i}}{2 {\left({δz}_{i}\right)}^{2}} C \left(x,z+{δz}_{i},τ\right)\end{matrix}$

### Discrétisation aux bords

Nous obtenons les équivalences suivantes pour les équations de bord : $\frac{C \left({x}_{0}+δx,z,t\right) - C \left({x}_{0}-δx,z,t\right)}{δx} = 0 ↔ C \left({x}_{0}-δx,z,t\right) = C \left({x}_{0}+δx,z,t\right)$ $\frac{C \left({x}_{4}+δx,z,t\right) - C \left({x}_{4}-δx,z,t\right)}{δx} = 0 ↔ C \left({x}_{4}-δx,z,t\right) = C \left({x}_{4}+δx,z,t\right)$ $\frac{C \left(x,{z}_{0}+δz,t\right) - C \left(x,{z}_{0}-δz,t\right)}{δz} = 0 ↔ C \left(x,{z}_{0}-δz,t\right) = C \left(x,{z}_{0}+δz,t\right)$ $\frac{C \left(x,{z}_{16}+δz,t\right) - C \left(x,{z}_{16}-δz,t\right)}{δz} = 0 ↔ C \left(x,{z}_{16}-δz,t\right) = C \left(x,{z}_{16}+δz,t\right)$

En substituant dans l'équation discrète de convection-diffusion :
Cas particulier du coin inférieur gauche (*x*₀, *z*₀) $\begin{matrix}\frac{C \left({x}_{0},{z}_{0},t+δt\right) - C \left({x}_{0}, {z}_{0}, t\right)}{δt} \\ = \left[\frac{- 2 {D}_{1}}{{\left(δx\right)}^{2}}\right] C \left({x}_{0}, {z}_{0}, τ\right) + \left[\frac{2 {D}_{1}}{{\left(δx\right)}^{2}}\right] C \left({x}_{0}+δx,{z}_{0},τ\right) + \left[\frac{- 2 {D}_{1}}{{\left({δz}_{1}\right)}^{2}}\right] C \left({x}_{0}, {z}_{0}, τ\right) \\ + \left[\frac{2 {D}_{1}}{{\left({δz}_{1}\right)}^{2}}\right] C \left({x}_{0},{z}_{0}+{δz}_{1},τ\right)\end{matrix}$

Cas particulier du bord inférieur (*xₖₓ, z*₀); *kₓ* ∈ {1,2,3,4} $\begin{matrix}\frac{C \left({x}_{{k}_{x}},{z}_{0},t+δt\right) - C \left({x}_{{k}_{x}}, {z}_{0}, t\right)}{δt} \\ = \left[\frac{{u}_{x} δx + 2 {D}_{1}}{2 {\left(δx\right)}^{2}}\right] C \left({x}_{{k}_{x}}-δx,{z}_{0},τ\right) + \left[\frac{- 2 {D}_{1}}{{\left(δx\right)}^{2}}\right] C \left({x}_{{k}_{x}}, {z}_{0}, τ\right) + \left[\frac{- {u}_{x} δx + 2 {D}_{1}}{2 {\left(δx\right)}^{2}}\right] C \left({x}_{{k}_{x}}+δx,{z}_{0},τ\right) \\ + \left[\frac{- 2 {D}_{1}}{{\left({δz}_{1}\right)}^{2}}\right] C \left({x}_{{k}_{x}}, {z}_{0}, τ\right) + \left[\frac{2 {D}_{1}}{{\left({δz}_{1}\right)}^{2}}\right] C \left({x}_{{k}_{x}},{z}_{0}+{δz}_{1},τ\right) ; {k}_{x} ∈ \left\{1, 2, 3, 4\right\}\end{matrix}$

Cas particulier du coin inférieur droit (*x*₄,*z*₀) $\begin{matrix}\frac{C \left({x}_{4},{z}_{0},t+δt\right) - C \left({x}_{4}, {z}_{0}, t\right)}{δt} \\ = \left[\frac{- 2 {D}_{1}}{{\left(δx\right)}^{2}}\right] C \left({x}_{4}, {z}_{0}, τ\right) + \left[\frac{2 {D}_{1}}{{\left(δx\right)}^{2}}\right] C \left({x}_{4}-δx,{z}_{0},τ\right) + \left[\frac{- 2 {D}_{1}}{{\left({δz}_{1}\right)}^{2}}\right] C \left({x}_{4}, {z}_{0}, τ\right) \\ + \left[\frac{2 {D}_{1}}{{\left({δz}_{1}\right)}^{2}}\right] C \left({x}_{4},{z}_{0}+{δz}_{1},τ\right)\end{matrix}$

Cas particulier du coin supérieur gauche (*x*₀,*z*₁₆) $\begin{matrix}\frac{C \left({x}_{0},{z}_{16},t+δt\right) - C \left({x}_{0}, {z}_{16}, t\right)}{δt} \\ = \left[\frac{- 2 {D}_{4}}{{\left(δx\right)}^{2}}\right] C \left({x}_{0}, {z}_{16}, τ\right) + \left[\frac{2 {D}_{4}}{{\left(δx\right)}^{2}}\right] C \left({x}_{0}+δx,{z}_{16},τ\right) + \left[\frac{- 2 {D}_{4}}{{\left({δz}_{4}\right)}^{2}}\right] C \left({x}_{0}, {z}_{16}, τ\right) \\ + \left[\frac{2 {D}_{4}}{{\left({δz}_{4}\right)}^{2}}\right] C \left({x}_{0},{z}_{16}-{δz}_{4},τ\right)\end{matrix}$

Cas particulier du bord supérieur (*xₖₓ,z*₁₆); *kₓ* ∈ {1,2,3,4} $\begin{matrix}\frac{C \left({x}_{{k}_{x}},{z}_{16},t+δt\right) - C \left({x}_{{k}_{x}}, {z}_{16}, t\right)}{δt} \\ = \left[\frac{{u}_{x} δx + 2 {D}_{4}}{2 {\left(δx\right)}^{2}}\right] C \left({x}_{{k}_{x}}-δx,{z}_{16},τ\right) + \left[\frac{- 2 {D}_{4}}{{\left(δx\right)}^{2}}\right] C \left({x}_{{k}_{x}}, {z}_{16}, τ\right) \\ + \left[\frac{- {u}_{x} δx + 2 {D}_{4}}{2 {\left(δx\right)}^{2}}\right] C \left({x}_{{k}_{x}}+δx,{z}_{16},τ\right) + \left[\frac{- 2 {D}_{4}}{{\left({δz}_{4}\right)}^{2}}\right] C \left({x}_{{k}_{x}}, {z}_{16}, τ\right) \\ + \left[\frac{2 {D}_{4}}{{\left({δz}_{4}\right)}^{2}}\right] C \left({x}_{{k}_{x}},{z}_{16}-{δz}_{4},τ\right) ; {k}_{x} ∈ \left\{1, 2, 3, 4\right\}\end{matrix}$

Cas particulier du coin supérieur droit (*x*₄,*z*₁₆) $\begin{matrix}\frac{C \left({x}_{4},{z}_{16},t+δt\right) - C \left({x}_{4}, {z}_{16}, t\right)}{δt} \\ = \left[\frac{- 2 {D}_{4}}{{\left(δx\right)}^{2}}\right] C \left({x}_{4}, {z}_{16}, τ\right) + \left[\frac{2 {D}_{4}}{{\left(δx\right)}^{2}}\right] C \left({x}_{4}-δx,{z}_{16},τ\right) + \left[\frac{- 2 {D}_{4}}{{\left({δz}_{4}\right)}^{2}}\right] C \left({x}_{4}, {z}_{16}, τ\right) \\ + \left[\frac{2 {D}_{4}}{{\left({δz}_{4}\right)}^{2}}\right] C \left({x}_{4},{z}_{16}-{δz}_{4},τ\right)\end{matrix}$

Cas particulier du bord gauche (*x*₀,*z_{kz}*) ; *k_{z}* ∈ {0,4, ... , 12,16} $\begin{matrix}\frac{C \left({x}_{0},{z}_{{k}_{z}},t+δt\right) - C \left({x}_{0}, {z}_{{k}_{z}}, t\right)}{δt} \\ = \left[\frac{- 2 {D}_{i}}{{\left(δx\right)}^{2}}\right] C \left({x}_{0}, {z}_{{k}_{z}}, τ\right) + \left[\frac{2 {D}_{i}}{{\left(δx\right)}^{2}}\right] C \left({x}_{0}+δx,{z}_{{k}_{z}},τ\right) + \frac{{D}_{i}}{{\left({δz}_{i}\right)}^{2}} C \left(x,{z}_{{k}_{z}}-{δz}_{i},τ\right) \\ + \frac{- 2 {D}_{i}}{{\left({δz}_{i}\right)}^{2}} C \left({x}_{0}, {z}_{{k}_{z}}, τ\right) + \frac{{D}_{i}}{{\left({δz}_{i}\right)}^{2}} C \left({x}_{0},{z}_{{k}_{z}}+{δz}_{i},τ\right) ; {k}_{z} ∈ \left\{0,4,…,12,16\right\}\end{matrix}$

Cas particulier du bord droit (*x*₄,*z_{kz}*) ; *k_{z}* ∈ {0, ... , 16} $\begin{matrix}\frac{\left({x}_{4},{z}_{{k}_{z}},t+δt\right) - C \left({x}_{4}, {z}_{{k}_{z}}, t\right)}{δt} \\ = \left[\frac{- 2 {D}_{i}}{{\left(δx\right)}^{2}}\right] C \left({x}_{4}, {z}_{{k}_{z}}, τ\right) + \left[\frac{2 {D}_{i}}{{\left(δx\right)}^{2}}\right] C \left({x}_{4}-δx,{z}_{{k}_{z}},τ\right) + \frac{{D}_{i}}{{\left({δz}_{i}\right)}^{2}} C \left({x}_{4},{z}_{{k}_{z}}-{δz}_{i},τ\right) \\ + \frac{- 2 {D}_{i}}{{\left({δz}_{i}\right)}^{2}} C \left({x}_{4}, {z}_{{k}_{z}}, τ\right) + \frac{{D}_{i}}{{\left({δz}_{i}\right)}^{2}} C \left({x}_{4},{z}_{{k}_{z}}+{δz}_{i},τ\right) ; {k}_{z} ∈ \left\{0,…,16\right\}\end{matrix}$

### Discrétisation aux interfaces

Selon le même principe que pour le modèle 1D, nous introduisons sur les points d'interface *z*_{1|2} et *z*_{2|3} et *z*_{3|4} les variables de concentration fictives correspondantes : *C*_{1|2} , *C*_{2|3} et *C*_{3|4}, cf. figure 11. *C*_{1|2} et *C*_{2|3} et *C*_{3|4} vont être éliminées du système par substitution.

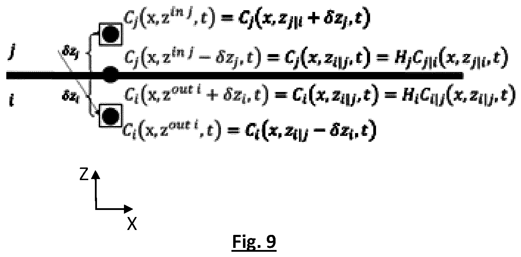

Dans la figure 9, nous illustrons la manière de discrétiser autour d'une interface qui sépare deux compartiments physiques.

Les notations z*^{out i}* et z*^{in j}* correspondent à *k_{z}*= 4, 8 ou 12 et « in » indique une entrée dans un milieu et « out » une sortie du milieu.

Nous obtenons pour la discrétisation des équations 2D-10 et 2D-11 : $\left\{\begin{matrix}{r}_{transpa i \left|j\right.} {D}_{i} \frac{{C}_{i} \left(x, {z}_{i \left|j\right.}, t\right) - {C}_{i} \left(x,{z}_{i \left|j\right.}-{δz}_{i},t\right)}{{δz}_{i}} = {D}_{j} \frac{{C}_{j} \left(x,{z}_{i \left|j\right.}+{δz}_{+},t\right) - {C}_{j} \left(x, {z}_{i \left|j\right.}, t\right)}{{δz}_{j}} \\ {C}_{i} \left(x, {z}_{i \left|j\right.}, t\right) = {H}_{i} {C}_{i \left|j\right.} \left(x, {z}_{i \left|j\right.}, t\right) \\ {C}_{j} \left(x, {z}_{j \left|i\right.}, t\right) = {H}_{j} {C}_{j \left|i\right.} \left(x, {z}_{j \left|i\right.}, t\right) \\ {C}_{i \left|j\right.} \left(x, {z}_{i \left|j\right.}, t\right) = {C}_{j \left|i\right.} \left(x, {z}_{j \left|i\right.}, t\right)\end{matrix}\right.$

En remplaçant la concentration déterminée par la loi d'Henry (*Cᵢ*(*x, z*_{*i*|}*ⱼ, t*), *Cⱼ*(*x, z*_{*j*|}*ᵢ, t*)) par celle de référence à l'interface *C*_{*j*|*i*}(*x, z*_{*j*|}*ᵢ, t),* $\begin{matrix}{r}_{transpa i \left|j\right.} \frac{{D}_{i}}{{δz}_{i}} {H}_{i} {C}_{i \left|j\right.} \left(x, {z}_{i \left|j\right.}, t\right) + \frac{{D}_{j}}{{δz}_{j}} {H}_{j} {C}_{i \left|j\right.} \left(x, {z}_{i \left|j\right.}, t\right) \\ = \frac{{D}_{j}}{{δz}_{j}} {C}_{j} \left(x,{z}_{i \left|j\right.}+{δz}_{j},t\right) + {r}_{transpa i \left|j\right.} \frac{{D}_{i}}{{δz}_{i}} {C}_{i} \left(x,{z}_{i \left|j\right.}-{δz}_{i},t\right)\end{matrix}$

Nous faisons la notation suivante : ${ζ}_{i \left|j\right.} = \frac{1}{{r}_{transpa i \left|j\right.} {H}_{i} \frac{{D}_{i}}{{δz}_{i}} + {H}_{j} \frac{{D}_{j}}{{δz}_{j}}}$

L'équation (2D-31) devient ${C}_{j \left|i\right.} \left(x, {z}_{j \left|i\right.}, t\right) = {C}_{i \left|j\right.} \left(x, {z}_{i \left|j\right.}, t\right) = {ζ}_{i \left|j\right.} \left(\frac{{D}_{j}}{{δz}_{j}}{{{C}_{j} \left(x,{z}_{i \left|j\right.}+{δz}_{j},t\right)}_{︸}}_{{C}_{j} \left(x, {z}^{in j}, t\right)}+{r}_{transpa i \left|j\right.}\frac{{D}_{i}}{{δz}_{i}}{{{C}_{i} \left(x,{z}_{i \left|j\right.}-{δz}_{i},t\right)}_{︸}}_{{C}_{i} \left(x, {z}^{out i}, t\right)}\right)$

Dans cette équation, nous reconnaissons les deux termes *Cⱼ*(*x, z*_{*i*|}*ⱼ + δzⱼ, t*) et *Cᵢ*(*x, z*_{*i*|}*ⱼ - δzᵢ*, *t*) comme étant respectivement les valeurs de *Cᵢ*(*x, z^{in j}, t*) et *Cᵢ*(*x, z^{out i}*, *t*).

Si nous nous plaçons au point juste avant l'interface (*Cᵢ*(*x, z^{out i}, t*) = *Cᵢ*(*x, z*_{*i*|*j*} - *δzᵢ, t*), la concentration au point précédent *Cᵢ*(*x,* z*^{out i}* - *δzᵢ, t*) est interne au milieu, la concentration au point suivant est placé sur l'interface c'est *Cᵢ*(*x,* z*^{out i}* + *δzᵢ, t*) qui est égale à *C*_{*i*|*j*}(*x, z*_{*i*|}*ⱼ, t*) : $\begin{matrix}{C}_{i} \left(x,{z}^{out i}+{δz}_{i},t\right) = {H}_{i} {C}_{i \left|j\right.} \left(x, {z}_{i \left|j\right.}, t\right) = {C}_{i} \left(x, {z}_{i \left|j\right.}, t\right) \\ = {H}_{i} {ζ}_{i \left|j\right.} \left(\frac{{D}_{j}}{{δz}_{j}}{C}_{j}\left(x,{z}_{i \left|j\right.}+{δz}_{j},t\right)+{r}_{transpa i \left|j\right.}\frac{{D}_{i}}{{δz}_{i}}{C}_{i}\left(x,{z}_{i \left|j\right.}-{δz}_{i},t\right)\right)\end{matrix}$

L'expression de la concentration *Cᵢ*(*x, z^{out i}* + *δzᵢ*, *t*) ainsi déterminée est intégrée dans l'équation discrète (2D-22) de convection-diffusion afin de calculer la dynamique dans le point z*^{out i}.* $\begin{matrix}\frac{C \left(x,{z}^{out i},t+δt\right) - C \left(x, {z}^{out i}, t\right)}{δt} \\ = \left[\frac{{u}_{x} δx + 2 {D}_{i}}{2 {\left(δx\right)}^{2}}\right] C \left(x-δx{, 2}^{out i},τ\right) + \left[\frac{- 2 {D}_{i}}{{\left(δx\right)}^{2}}C\left(x, {z}^{out i}, τ\right)\right] \\ + \left[\frac{- {u}_{x} δx + 2 {D}_{i}}{2 {\left(δx\right)}^{2}}\right] C \left(x+δx,{z}^{out i}+τ\right) + \frac{2 {D}_{i}}{2 {\left({δz}_{i}\right)}^{2}} C \left(x,{z}^{out i}-{δz}_{i},τ\right) + \frac{- 2 {D}_{i}}{{\left({δz}_{i}\right)}^{2}} C \left(x, {z}^{out i}, τ\right) \\ + \frac{2 {D}_{i}}{2 {\left({δz}_{i}\right)}^{2}} {H}_{i} {ζ}_{i \left|j\right.} \left(\frac{{D}_{j}}{{δz}_{j}}{C}_{j}\left(x,{z}_{i \left|j\right.}+{δz}_{j},t\right)+{r}_{transpa i \left|j\right.}\frac{{D}_{i}}{{δz}_{i}}{C}_{i}\left(x,{z}_{i \left|j\right.}-{δz}_{i},t\right)\right)\end{matrix}$

Soit encore : $\begin{matrix}\frac{C \left(x,{z}^{out i},t+δt\right) - C \left(x, {z}^{out i}, t\right)}{δt} \\ = \left[\frac{{u}_{x} δx + 2 {D}_{i}}{2 {\left(δx\right)}^{2}}\right] C \left(x-δx,{z}^{out i},τ\right) + \left[\frac{- 2 {D}_{i}}{{\left(δx\right)}^{2}}\right] C \left(x, {z}^{out i}, τ\right) \\ + \left[\frac{- {u}_{x} δx + 2 {D}_{i}}{2 {\left(δx\right)}^{2}}\right] C \left(x+δx,{z}^{out i},τ\right) + \left[\frac{2 {D}_{i}}{2 {\left({δz}_{i}\right)}^{2}}\right] C \left(x,{z}^{out i}-{δz}_{i},τ\right) \\ + \left[\frac{- 2 {D}_{i}}{{\left({δz}_{i}\right)}^{2}}+{r}_{transpa i \left|j\right.}{λ}_{i \left|j\right.}\frac{{D}_{i}}{{δz}_{i}}\right] C \left(x, {z}^{out i}, τ\right) + \left[{λ}_{i \left|j\right.}\frac{{D}_{j}}{{δz}_{j}}\right] C \left(x, {z}^{in j}, t\right)\end{matrix}$

Où : ${λ}_{i \left|j\right.} = \frac{2 D}{2 {\left({δz}_{i}\right)}^{2}} {H}_{i} {ζ}_{i \left|j\right.}$

Si nous nous situons au point juste après l'interface *Cⱼ*(*z^{in j}, t*) *= Cⱼ*(*z*_{*j*|}*ᵢ + δzⱼ, t*), la concentration au point suivant *Cᵢ*(*x, z^{in j} + δzⱼ, t*) est interne au milieu, la concentration au point précédent est situé sur l'interface *Cⱼ*(*x,* z*^{in j}* - *δzⱼ, t*) est *Cⱼ*(*x, z*_{*j*|}*ᵢ, t*) qui est égale à *Cⱼ*(*z*_{*j*|}*ᵢ, t*) : $\begin{matrix}{C}_{j} \left(x,{z}^{in j}-{δz}_{j},t\right) = {C}_{j} \left(x, {z}_{j \left|i\right.}, t\right) = {H}_{j} {C}_{j \left|i\right.} \left(x, {z}_{j \left|i\right.}, t\right) \\ = {H}_{j} {ζ}_{i \left|j\right.} \left(\frac{{D}_{j}}{{δz}_{j}}{C}_{j}\left(x, {z}^{in j}, t\right)+{r}_{transpa i \left|j\right.}\frac{{D}_{i}}{{δz}_{i}}{C}_{i}\left(x, {z}^{out i}, t\right)\right)\end{matrix}$

L'expression de la concentration *C*(z*^{in j}* - *δzⱼ, t*) est intégrée dans l'équation de convection-diffusion discrète (2D-22) afin de calculer la dynamique dans le point z*^{in j}* : $\begin{matrix}\frac{C \left(x,{z}^{in j},t+δt\right) - C \left(x, {z}^{in j}, t\right)}{δt} \\ = \left[\frac{{u}_{x} δx + 2 {D}_{i}}{2 {\left(δx\right)}^{2}}\right] C \left(x-δx,{z}^{in j},τ\right) + \left[\frac{- 2 {D}_{i}}{{\left(δx\right)}^{2}}\right] C \left(x, {z}^{in j}, τ\right) + \left[\frac{- {u}_{x} δx + 2 {D}_{i}}{2 {\left(δx\right)}^{2}}\right] C \left(x+δx,{z}^{in j},τ\right) \\ + \frac{2 {D}_{j}}{2 {\left({δz}_{j}\right)}^{2}} {H}_{j} {ζ}_{i \left|j\right.} \left(\frac{{D}_{j}}{{δz}_{j}}{C}_{j}\left(x, {z}^{in j}, t\right)+{r}_{transpa i \left|j\right.}\frac{{D}_{i}}{{δz}_{i}}{C}_{i}\left(x, {z}^{out i}, t\right)\right) - \frac{2 {D}_{j}}{{\left({δz}_{j}\right)}^{2}} C \left(x, {z}^{in j , τ}\right) \\ + \frac{2 {D}_{j}}{2 {\left({δz}_{j}\right)}^{2}} C \left(x,{z}^{in j}+{δz}_{j},τ\right)\end{matrix}$

Soit encore : $\begin{matrix}\frac{C \left(x,{z}^{in j},t+δt\right) - C \left(x, {z}^{in j}, t\right)}{δt} \\ = \left[\frac{{u}_{x} δx + 2 {D}_{i}}{2 {\left(δx\right)}^{2}}\right] C \left(x-δx,{z}^{in j},τ\right) + \left[\frac{- 2 {D}_{i}}{{\left(δx\right)}^{2}}\right] C \left(x, {z}^{in j}, τ\right) + \left[\frac{- {u}_{x} δx + 2 {D}_{i}}{2 {\left(δx\right)}^{2}}\right] C \left(x+δx,{z}^{in j},τ\right) \\ + \left[\frac{{λ}_{j \left|i\right.} {D}_{j}}{{δz}_{j}}-\frac{2 {D}_{j}}{{\left({δz}_{j}\right)}^{2}}\right] {C}_{j} \left(x, {z}^{in j}, t\right) + \left[{r}_{transpa i \left|j\right.}{λ}_{j \left|i\right.}\frac{{D}_{i}}{{δz}_{i}}\right] {C}_{i} \left(x, {z}^{out i}, t\right) \\ + \left[\frac{2 {D}_{j}}{2 {\left({δz}_{j}\right)}^{2}}\right] C \left(x,{z}^{in j}+{δz}_{j},τ\right)\end{matrix}$

Où : ${λ}_{j \left|i\right.} = \frac{2 {D}_{j}}{2 {\left({δz}_{j}\right)}^{2}} {H}_{j} {ζ}_{i \left|j\right.}$

Les équations précédentes peuvent être écrites sous une forme matricielle en définissant une matrice opérateur différentiel spatial ***F*₀** qui représente un opérateur avec des éléments qui sont les termes entre crochets de ces équations: ${c}_{0} \left(x,z,t+δt\right) - {c}_{0} \left(x, z, t\right) = δt ⋅ {F}_{0} ⋅ {c}_{0} \left(x, z, τ\right)$

Cette matrice opérateur différentiel spatial définit la discrétisation temporelle :
Si *τ = t* nous utilisons un schéma d'intégration dans le temps d'Euler explicite : ${c}_{0} \left(x,z,t+δt\right) - {c}_{0} \left(x, z, t\right) = δt ⋅ {F}_{0} ⋅ {c}_{0} \left(x, z, t\right)$

Soit encore : ${c}_{0} \left(x,z,t+δt\right) = \left(I+δt⋅{F}_{0}\right) ⋅ {c}_{0} \left(x, z, t\right)$

Si *τ = t + δt* nous utilisons un schéma d'intégration dans le temps d'Euler implicite : ${c}_{0} \left(x,z,t+δt\right) - {c}_{0} \left(x, z, t\right) = δt ⋅ {F}_{0} ⋅ {c}_{0} \left(x,z,t+δt\right)$

Soit encore : $\left(I-δt⋅{F}_{0}\right) ⋅ {c}_{0} \left(x,z,t+δt\right) = {c}_{0} \left(x, z, t\right)$

Les matrices opérateur différentiel spatial ***F*₀** s'expriment ainsi en isolant les termes liés respectivement à la convection, à la diffusion, et aux conditions aux interfaces et aux bords : ${F}_{0} = {F}_{0 conv} + {F}_{0 dif} + {F}_{0 int}$
***F*_{0 *conv*}** regroupe les termes de l'équation liés à la convection,
***F*_{0 *dif*}** regroupe les termes de l'équation liés à la diffusion ;
***F*_{0 *int*}** regroupe les termes de l'équation liés aux interfaces et aux bords ;

Les points de discrétisation sont balayés dans la direction x puis dans la direction z pour construire les matrices. Nous notons *k_{F}* les indices des éléments de cette matrice.

Si la position (*x, z*) a pour indice (*k_{F}*; *k_{F}* =∈ {0, . . , *N -* 1}) dans ces matrices selon ce balayage en x puis en z, les positions suivantes auront les indices suivants :

| | |
|---|---|
| - (*x* - *δx,z*) | indice (*k_{F} -* 1) |
| - (*x, z*) | indice (*k_{F}*) |
| - (*x* + *δx,z*) | indice (*k_{F} +* 1) |
| - (*x, zᵢ* - *δzᵢ*) | indice (*k_{F} - Nₓ*) |
| - (*x*, *zᵢ* + *δzᵢ*) | indice (*k_{F} + Nₓ*) |

Les position des bords sont définies par les points (*x, z*) ayant dans ces matrices les indices (*k_{F}*) suivants :

| | |
|---|---|
| - bord inférieur | indice *k_{F} =* (*kₓ*); *kₓ* =∈ {0,.., (*Nx* - 1)} |
| - bord supérieur | indice *k_{F} =* (*kₓ +* (*N_{z} -* 1). *Nₓ*); *kₓ* =∈ {0,..,(*Nx* - 1)} |
| - bord gauche | indice *k_{F} =* (0 *+ k_{z}.Nₓ*); *k_{z}* =∈ {1,..,*Nz* - 2} |
| - bord droit | indice *k_{F} =* ((*Nₓ -* 1) *+ k_{z}.Nₓ*); *k_{z}* =∈ {1,..,*Nz* - 2} |

Les position des interfaces sont définies par les points (*x, z*) ayant dans ces matrices les indices (*k_{F}*) suivants :

| | |
|---|---|
| - première interface | *k_{F}* = (*kₓ +* (*N*_{*z*,1} - 1).*Nₓ*); *kₓ* =∈ {0,..,(*Nx* - 1)} |
| - deuxième interface | *k_{F}* = (*kₓ +* (*N*_{*z*,1} - 1 + *N*_{*z*,2} - 1).*Nₓ*); *kₓ* =∈ {0,..,(*Nx* - 1)} |
| - troisième interface | *k_{F}* = (*kₓ +* (*N*_{*z*,1} - 1 + *N*_{*z,*2} - 1 + *N*_{*z*,3} - 1).*Nₓ*); *kₓ* =∈ {0,..,(*Nx* - 1)} |

Ces matrices sont creuses, nous notons *f_{kF,kF}*, l'élément de matrice à la position *k_{F}, k_{F}'.*

***F*_{0 *conv*}** est construite de la manière suivante : ${f}_{{k}_{F} , {k}_{F} - 1} = \frac{{u}_{x}}{2 δx}$ ${f}_{{k}_{F} , {k}_{F} + 1} = - \frac{{u}_{x}}{2 δx}$ les autres termes de ***F*_{0 *conv*}** prennent une valeur nulle.

***F*_{0 *dif*}** est construite de la manière suivante : ${f}_{{k}_{F} , {k}_{F}} = - 2 {D}_{i} \left[\frac{1}{{\left(δx\right)}^{2}}+\frac{1}{{\left({δz}_{i}\right)}^{2}}\right]$ ${f}_{{k}_{F} , {k}_{F} - 1} = \frac{{D}_{i}}{{\left(δx\right)}^{2}}$ ${f}_{{k}_{F} , {k}_{F} + 1} = \frac{{D}_{i}}{{\left(δx\right)}^{2}}$ ${f}_{{k}_{F} , {k}_{F} - {N}_{x}} = \frac{{D}_{i}}{{\left({δz}_{i}\right)}^{2}}$ ${f}_{{k}_{F} , {k}_{F} + {N}_{x}} = \frac{{D}_{i}}{{\left({δz}_{i}\right)}^{2}}$ les autres termes de ***F*_{0 *dif*}** prennent une valeur nulle.

***F*_{0 *int*}** est construite de la manière suivante :
Pour les échantillons en-dessous de l'interface : (*k_{F} = k_{Finterface} - Nₓ*) ${k}_{F} = \left({k}_{x}+\left({N}_{z , 1}-2\right).{N}_{x}\right) ; {k}_{x} = ∈ \left\{0,..,\left(Nx-1\right)\right\}$ ${k}_{F} = \left({k}_{x}+\left({N}_{z , 1}-1+{N}_{z , 2}-2\right).{N}_{x}\right) ; {k}_{x} = ∈ \left\{0,..,\left(Nx-1\right)\right\}$ ${k}_{F} = \left({k}_{x}+\left({N}_{z , 1}-1+{N}_{z , 2}-1+{N}_{z , 3}-2\right).{N}_{x}\right) ; {k}_{x} = ∈ \left\{0,..,\left(Nx-1\right)\right\}$ ${f}_{{k}_{F} , {k}_{F} - 1} = \frac{{u}_{x} δx + 2 {D}_{i}}{2 {\left(δx\right)}^{2}}$ ${f}_{{k}_{F} , {k}_{F}} = \frac{- 2 {D}_{i}}{{\left(δx\right)}^{2}} + \frac{- 2 {D}_{i}}{{\left({δz}_{i}\right)}^{2}} + {r}_{transpa i \left|j\right.} {λ}_{i \left|j\right.} \frac{{D}_{i}}{{δz}_{i}}$ ${f}_{{k}_{F} , {k}_{F} + 1} = \frac{- {u}_{x} δx + 2 {D}_{i}}{2 {\left(δx\right)}^{2}}$ ${f}_{{k}_{F} , {k}_{F} - {N}_{x}} = \frac{2 {D}_{i}}{2 {\left({δz}_{i}\right)}^{2}}$ ${f}_{{k}_{F} , {k}_{F} + 2 {N}_{x}} = {λ}_{i \left|j\right.} \frac{{D}_{j}}{{δz}_{j}}$ ${f}_{{k}_{F} - {N}_{x} , {k}_{F} + {N}_{x}} = {λ}_{i \left|j\right.} \frac{{D}_{j}}{{δz}_{j}}$

Pour les échantillons au-dessus de l'interface (*k_{F} = K_{Finterface} + Nₓ*) : ${k}_{F} = \left({k}_{x}+\left({N}_{z , 1}\right).{N}_{x}\right) ; {k}_{x} = ∈ \left\{0,..,\left(Nx-1\right)\right\}$ ${k}_{F} = \left({k}_{x}+\left({N}_{z , 1}-1+{N}_{z , 2}\right).{N}_{x}\right) ; {k}_{x} = ∈ \left\{0,..,\left(Nx-1\right)\right\}$ ${k}_{F} = \left({k}_{x}+\left({N}_{z , 1}-1+{N}_{z , 2}-1+{N}_{z , 3}\right).{N}_{x}\right) ; {k}_{x} = ∈ \left\{0,..,\left(Nx-1\right)\right\}$ ${f}_{{k}_{F} , {k}_{F} - 1} = \frac{{u}_{x} δx + 2 {D}_{i}}{2 {\left(δx\right)}^{2}}$ ${f}_{{k}_{F} , {k}_{F}} = \frac{- 2 {D}_{i}}{{\left(δx\right)}^{2}} - \frac{2 {D}_{j}}{{\left({δz}_{j}\right)}^{2}} + \frac{{λ}_{i \left|j\right.} {D}_{j}}{{δz}_{j}}$ ${f}_{{k}_{F} , {k}_{F} + 1} = \frac{- {u}_{x} δx + 2 {D}_{i}}{2 {\left(δx\right)}^{2}}$ ${f}_{{k}_{F} , {k}_{F} - 2 {N}_{x}} = {r}_{transpa \left.i\right| j} {λ}_{\left.j\right| i} \frac{{D}_{i}}{{δz}_{i}}$ ${f}_{{k}_{F} , {k}_{F} + {N}_{x}} = \frac{2 {D}_{j}}{2 {\left({δz}_{j}\right)}^{2}}$

Où *ζ*_{*i*|*j*}, et *λ*_{*i*|*j*} et *λ*_{*j*|*i*} ont été définis plus haut (équations 2D-33 et 2D-37 et 2D-41).

Pour les bords : $\left\{\begin{matrix}{f}_{0 , 0} = \frac{- 2 {D}_{1}}{{\left(δx\right)}^{2}} + \frac{- 2 {D}_{1}}{{\left({δz}_{1}\right)}^{2}} \\ {f}_{0 , 1} = \frac{2 {D}_{1}}{{\left(δx\right)}^{2}} \\ {f}_{0 , {N}_{x}} = \frac{2 {D}_{1}}{{\left({δz}_{1}\right)}^{2}}\end{matrix}\right. \left\{\begin{matrix}{f}_{4 , 4} = \frac{- 2 {D}_{1}}{{\left(δx\right)}^{2}} + \frac{- 2 {D}_{1}}{{\left({δz}_{1}\right)}^{2}} \\ {f}_{4 , 3} = \frac{2 {D}_{1}}{{\left(δx\right)}^{2}} \\ {f}_{4 , 4 + {N}_{x}} = \frac{2 {D}_{1}}{{\left({δz}_{1}\right)}^{2}}\end{matrix}\right.$ $\left\{\begin{matrix}{f}_{\left({N}_{z}-1\right) . {N}_{x} , \left({N}_{z}-1\right) . {N}_{x}} = \frac{- 2 {D}_{4}}{{\left(δx\right)}^{2}} + \frac{- 2 {D}_{4}}{{\left({δz}_{4}\right)}^{2}} \\ {f}_{\left({N}_{z}-1\right) . {N}_{x} , \left({N}_{z}-1\right) . {N}_{x} + 1} = \frac{2 {D}_{4}}{{\left(δx\right)}^{2}} \\ {f}_{\left({N}_{z}-1\right) . {N}_{x} , \left({N}_{z}-2\right) . {N}_{x}} = \frac{2 {D}_{4}}{{\left({δz}_{4}\right)}^{2}}\end{matrix}\right. \left\{\begin{matrix}{f}_{4 + \left({N}_{z}-1\right) . {N}_{x} , 4 + \left({N}_{z}-1\right) . {N}_{x}} = \frac{- 2 {D}_{4}}{{\left(δx\right)}^{2}} + \frac{- 2 {D}_{4}}{{\left({δz}_{4}\right)}^{2}} \\ {f}_{4 + \left({N}_{z}-1\right) . {N}_{x} , 3 + \left({N}_{z}-1\right) . {N}_{x}} = \frac{2 {D}_{4}}{{\left(δx\right)}^{2}} \\ {f}_{4 + \left({N}_{z}-1\right) . {N}_{x} , 4 + \left({N}_{z}-2\right) . {N}_{x}} = \frac{2 {D}_{4}}{{\left({δz}_{4}\right)}^{2}}\end{matrix}\right.$ ${k}_{F} = \left({k}_{x}\right) ; {k}_{x} = ∈ \left\{1,…,\left(Nx-2\right)\right\}$ $\left\{\begin{matrix}{f}_{{k}_{F} , {k}_{F} - 1} = \frac{{u}_{x} δx + 2 {D}_{1}}{2 {\left(δx\right)}^{2}} \\ {f}_{{k}_{F} , {k}_{F}} = \frac{- 2 {D}_{1}}{{\left(δx\right)}^{2}} + \frac{- 2 {D}_{1}}{{\left({δz}_{1}\right)}^{2}} \\ {f}_{{k}_{F} , {k}_{F} + 1} = \frac{- {u}_{x} δx + 2 {D}_{1}}{2 {\left(δx\right)}^{2}} \\ {f}_{{k}_{F} , {k}_{F} + {N}_{x}} = \frac{2 {D}_{1}}{{\left({δz}_{1}\right)}^{2}}\end{matrix}\right.$ ${k}_{F} = \left({k}_{x}+\left({N}_{z}-1\right).{N}_{x}\right) ; {k}_{x} = ∈ \left\{1,…,\left(Nx-2\right)\right\}$ $\left\{\begin{matrix}{f}_{{k}_{F} , {k}_{F} - 1} = \frac{{u}_{x} δx + 2 {D}_{4}}{2 {\left(δx\right)}^{2}} \\ {f}_{{k}_{F} , {k}_{F}} = \frac{- 2 {D}_{4}}{{\left(δx\right)}^{2}} + \frac{- 2 {D}_{4}}{{\left({δz}_{1}\right)}^{2}} \\ {f}_{{k}_{F} , {k}_{F} + 1} = \frac{- {u}_{x} δx + 2 {D}_{4}}{2 {\left(δx\right)}^{2}} \\ {f}_{{k}_{F} , {k}_{F} - {N}_{x}} = \frac{2 {D}_{4}}{{\left({δz}_{4}\right)}^{2}}\end{matrix}\right.$ ${k}_{F} = \left(0+{k}_{z}.{N}_{x}\right) ; {k}_{z} = ∈ \left\{1,…,Nz-2\right\}$ $\left\{\begin{matrix}{f}_{{k}_{F} , {k}_{F}} = \frac{- 2 {D}_{i}}{{\left(δx\right)}^{2}} + \frac{- 2 {D}_{i}}{{\left({δz}_{i}\right)}^{2}} \\ {f}_{{k}_{F} , {k}_{F} + 1} = \frac{2 {D}_{i}}{{\left(δx\right)}^{2}} \\ {f}_{{k}_{F} , {k}_{F} - {N}_{x}} = \frac{- 2 {D}_{i}}{{\left({δz}_{i}\right)}^{2}} \\ {f}_{{k}_{F} , {k}_{F} + {N}_{x}} = \frac{{D}_{i}}{{\left({δz}_{i}\right)}^{2}}\end{matrix}\right.$ ${k}_{F} = \left(\left({N}_{x}-1\right)+{k}_{z}.{N}_{x}\right) ; {k}_{z} = ∈ \left\{1,..,Nz-2\right\}$ $\left\{\begin{matrix}{f}_{{k}_{F} , {k}_{F}} = \frac{- 2 {D}_{i}}{{\left(δx\right)}^{2}} + \frac{- 2 {D}_{i}}{{\left({δz}_{i}\right)}^{2}} \\ {f}_{{k}_{F} , {k}_{F} - 1} = \frac{2 {D}_{i}}{{\left(δx\right)}^{2}} \\ {f}_{{k}_{F} , {k}_{F} - {N}_{x}} = \frac{{D}_{i}}{{\left({δz}_{i}\right)}^{2}} \\ {f}_{{k}_{F} , {k}_{F} + {N}_{x}} = \frac{{D}_{i}}{{\left({δz}_{i}\right)}^{2}}\end{matrix}\right.$

Les autres termes de ***F*_{0 *int*}** prennent une valeur nulle.

### Problème direct restreint

Pour décrire le modèle direct de transport nous n'utilisons que les termes situés à l'intérieur strict des milieux en z en ne tenant pas compte des 3x(*Nₓ*) points qui correspondent aux interfaces sang/peau, peau/mes, mes/col et les points aux bords (latéraux, supérieur et inférieur). Nous avons décrit au-dessus comment éliminer des équations les points situés au niveau exact des interfaces par des points avant ou après l'interface. Les points de la matrice ***F*_{0 *int*}** correspondant aux interfaces sont éliminés des matrices.

Nous définissons une nouvelle matrice ***F*** de dimension (*Nₑₜₐₜ*)dans laquelle les points des interfaces ont été supprimés. Soit les points d'indices *k_{F}* définis au dessus. $F = {F}_{conv} + {F}_{dif} + {F}_{int}$

Nous définissons une matrice de commande G qui comprend les valeurs d'entrée connues.

G est une matrice à deux colonnes de taille (*Nₑₜₐₜ*, 2) de la forme (cas de l'évacuation transverse) : $G = \left\{\begin{matrix}{g}_{{k}_{G} , 1} ; {k}_{G} = \left(0+{k}_{z}.{N}_{k}\right) ; {k}_{z} = ∈ \left\{1, 2, 3\right\} \\ {g}_{{k}_{G} , 2} ; {k}_{G} = \left(0+\left({N}_{z , 1}+{N}_{z , 2}+{N}_{z , 3}-6+{k}_{z}\right).{N}_{x}\right) ; {k}_{z} = ∈ \left\{1, 2, 3\right\} \\ 0 pour tous les autres termes\end{matrix}\right.$

Où ${g}_{{k}_{G} , 1} = \frac{{u}_{x}^{sang}}{{δx}^{sang}}$ et ${g}_{{k}_{G} , 2} = \frac{{u}_{x}^{col}}{δx}$.

***q*** est le vecteur contenant les entrées exogènes dans le système. Ce sont la concentration initiale introduite dans le système dans la phase liquide *C^{in Sang}* d'une part, et la concentration du CO₂ dans l'air ambiant *C^{in Col Air}* d'autre part. $q = \left[\begin{matrix}{C}^{in Sang} \\ {C}^{in Col Air}\end{matrix}\right]$

Les données de concentration sont générées à l'aide d'un modèle de signal suivant la relation de récursivité : $A {c}_{{k}_{t} + 1} = {c}_{{k}_{t}} + G {q}_{{k}_{t} + 1}$

Où ***A*** est la matrice de transition obtenue à partir de la discrétisation temporelle implicite et ***c** -* le vecteur d'état de dimension *Nₑₜₐₜ* $A = I - F ⋅ δt$

Dans le cas de l'évacuation transverse à contre-courant, l'entrée d'air se fait par le côté opposé, la matrice G s'exprime ainsi : $G = \left\{\begin{matrix}{g}_{{k}_{G} , 1} ; {k}_{G} = \left(0+{k}_{z}.{N}_{k}\right) ; {k}_{z} = ∈ \left\{1, 2, 3\right\} \\ {g}_{{k}_{G} , 2} ; {k}_{G} = \left(0+\left({N}_{z , 1}+{N}_{z , 2}+{N}_{z , 3}-6+{k}_{z}\right).{N}_{x}\right) ; {k}_{z} = ∈ \left\{1, 2, 3\right\} \\ 0 pour tous les autres termes\end{matrix}\right.$

Où ${g}_{{k}_{G} , 1} = \frac{{u}_{x}^{sang}}{{δx}^{sang}}$ et ${g}_{{k}_{G} , 2} = \frac{- {u}_{x}^{col}}{δx}$.

L'algorithme de Kalman nous permet d'estimer de manière linéaire une variable d'intérêt, ici *C^{in Sang}*, en cherchant à minimiser l'erreur quadratique moyenne entre la valeur réelle et la valeur estimée. Il repose sur la formulation d'un modèle d'espace-état en associant une équation d'évolution des états, et une équation d'observation qui exprime le lien entre les mesures et les états. $c \left[{k}_{t}+1\right] = Fc \left[{k}_{t}\right] + Gq \left[{k}_{t}+1\right] + w \left[{k}_{t}+1\right]$ $y \left({k}_{t}\right) = Hc \left({k}_{t}\right) + v \left({k}_{t}\right)$

*kₜ* représente l'indice de discrétisation temporelle.

Le vecteur *w* décrit le bruit de modèle sur l'équation d'évolution de l'état, de matrice de variance-covariance ***Q***.

A travers la matrice d'observation ***H*,** nous définissons l'observable du vecteur d'état ***c*.** Dans le cas spécifique de notre problème, nous ne disposons que d'une observable pour le vecteur d'état, tous les autres variables de concentrations étant cachées, la matrice se réduit à un vecteur transposé. $H = {h}^{t}$

Le vecteur ***v*** décrit le bruit d'observation de matrice de variance-covariance ***R*.**

Nous devons augmenter le vecteur d'état avec la variable recherchée *C_{in sang}* de la même manière qu'en 1D.

La variable qu'on cherche à estimer *C_{in sang}*, se trouve dans le vecteur ***c̃****ₖ* à la position *x*₀, *z*₀. Elle est liée à la variable d'état suivante *C*_{*x*1,*z*1} par les termes de l'équation de transport. Nous supposons que le signal peut être modélisé par un modèle autorégressif d'ordre un défini par la relation de récurrence suivante : $\frac{\partial }{\partial t} {C}^{in sang} = {φC}^{in sang} + {w}^{in sang}$ sous forme discrète: $\left(1-φ\right) {C}_{in sang} \left[{k}_{t}+1\right] = δt {C}_{in sang} \left[{k}_{t}\right] + w \left[{k}_{t}+1\right]$

Où *φ* est un paramètre du modèle. Il faut interpréter *φ* comme un paramètre de régularité sur le signal.
Si *φ* = 0, la dérivée temporelle du signal en entrée est supposée nulle. Il n'y a pas de variation.
Si *φ >* 0 la dérivée du signal en entrée est supposée positive pour une concentration *C_{in sang}* positive. Cela favorise l'accroissement du signal, mais cela va pénaliser sa décroissance.

Nous définissons un système augmenté en associant le modèle physique du système décrit précédemment avec ce modèle de signal. $\left\{\begin{matrix}\frac{\partial }{\partial t} c = Fc + Gq + w \\ \frac{\partial }{\partial t} {C}^{in sang} = {φC}^{in sang} + {w}^{in sang}\end{matrix}\right.$

L'équation d'état du système augmenté s'écrit sous la forme suivante : $\frac{\partial }{\partial t} = {c}^{˜} = {F}^{˜} {c}^{˜} + {G}^{˜} {q}^{˜} + {w}^{˜}$

La discrétisation de manière implicite fournit l'équation suivante : ${A}^{˜} {{c}^{˜}}_{{k}_{t} + 1} = {{c}^{˜}}_{{k}_{t}} + {G}^{˜} {q}_{{k}_{t} + 1} + {{w}^{˜}}_{{k}_{t} + 1}$

Avec : ${A}^{˜} = \left[\begin{matrix}φ & 0 \\ {g}_{0 , 1} & \\ 0 & \\ 0 & \left[A\right] \\ 0 & \\ 0 & \end{matrix}\right]$ ${c}^{˜} = \left[\begin{matrix}{C}^{in S ang} \\ {C}_{{k}_{F}}\end{matrix}\right] ; {k}_{F} = 1 : {N}_{etat}$

***w̃***[*k*]~ 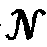 (**0**, ***Q̃***) est le bruit qui intègre les erreurs liées au modèle de dimension *N'x*1. où 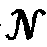 (**0**, ***Q̃***), est une loi normale vectorielle de moyenne nulle pour chaque composante et de matrice de variance-covariance ***Q̃***. ${{σ}^{˜}}_{w}^{2}$ la variance du bruit du modèle décrivant les erreurs liées au modèle commune à chaque composante du vecteur bruit ***w̃*** pour chaque instant *k**ₜ**.* La matrice de variance-covariance ***Q̃*** vaut ${Q}^{˜} = {{σ}^{˜}}_{w}^{2} . I$

***G̃*** la matrice de commande ne porte plus que sur *C^{in col air}* (suppression de la première colonne). ${G}^{˜} = \left[\begin{matrix}0 \\ ⋯ \\ 0 \\ {g}_{{k}_{G} , 2} \\ 0 \\ ⋯ \\ 0\end{matrix}\right]$ ${k}_{G} = \left(0+\left({N}_{z , 1}+{N}_{z , 2}+{N}_{z , 3}-6+{k}_{z}\right).{N}_{x}\right) ; {k}_{z} = ∈ \left\{1, 2, 3\right\}$ ${g}_{{k}_{G} , 2} = \frac{{u}_{x}^{col}}{δx}$

Comme indiqué ci-dessus, dans le cas de l'évacuation transverse à contre-courant : ${k}_{G} = \left(0+\left({N}_{z , 1}+{N}_{z , 2}+{N}_{z , 3}-6+{k}_{z}\right).{N}_{x}+\left({N}_{x}-1\right)\right) ; {k}_{z} = ∈ \left\{1, 2, 3\right\}$ ${g}_{{k}_{G} , 2} = \frac{- {u}_{x}^{col}}{δx}$

### Algorithme de filtrage de Kalman.

- ***c̃***_{*kt,kt*-1} est le vecteur d'état augmenté à l'instant *kₜ* prédit à l'instant *kₜ* - 1 précédent
- ***c̃****_{kt,kt}* est le vecteur d'état augmenté à l'instant *kₜ* calculé à l'instant *kₜ*
- ***c̃***_{*kt*+1,*kt*} est le vecteur d'état augmenté à l'instant *kₜ* + 1 prédit à l'instant *kₜ*
- ***P***_{***kt**,**kt***-1} est la matrice de covariance de l'erreur à l'instant *kₜ* prédite à l'instant *kₜ* - 1 précédent
- ***P**_{**kt**,**kt**}* est la matrice de covariance de l'erreur à l'instant *kₜ* calculée à l'instant *kₜ*
- ***P*_{*kt+*1,*kt*}** est la matrice de covariance de l'erreur à l'instant *kₜ* + 1 prédite à l'instant *kₜ*
- ***Kₖₜ*** est le gain de Kalman calculé à l'instant *kₜ*
- ***y**ₖₜ* est la nouvelle mesure observée à l'instant *kₜ*
- ***Ã*** est la matrice de transition appliquée à l'état ***c̃***_{*kt*+1}
- ***G̃*** est la matrice de commande appliquée à l'entrée ***q̃*** du système augmenté
- ***H̃*** est La matrice d'observation augmentée
- ***Q̃*** est la matrice de covariance du bruit du système restreint augmenté
- R est la matrice de covariance du bruit d'observation

Le problème à résoudre se résume à obtenir des relations récurrentes pour l'estimation du vecteur d'état ***c̃****_{**kt**,**kt**}* en fonction du vecteur d'état précédent ***c̃***_{***kt*-1***,****kt*-1**} et de la nouvelle observation *yₖₜ*. Deux étapes sont nécessaires pour résoudre ce problème :
#1 L'étape de correction et de mise à jour du vecteur d'état et de la matrice de covariance à partir des équations d'observation et du vecteur d'état et de la matrice de covariance estimés à l'étape de prédiction précédente.
#2 L'étape de prédiction du vecteur d'état et de la matrice de covariance à l'instant suivant à partir du vecteur d'état et de la matrice de covariance à l'instant précédent.

Ces deux étapes sont précédées d'une initialisation :
#0 On suppose connu l' état du système ***c*_{0,0}** à l'instant *kₜ* = 0 et la matrice désignant la covariance de l'erreur ***P*_{0,0}**.

Le vecteur d'état à l'instant *kₜ* = 0 est initialisé par le vecteur nul : ***c̃*_{0,0} = 0** (de dimensions *Nₑₜₐₜx*1) et la matrice désignant la covariance de l'erreur par la matrice identité (de dimensions *NₑₜₐₜxNₑₜₐₜ*)

L'algorithme est arrêté quand il n'y a plus de nouvelles mesures qui sont réalisées.

Nous présentons maintenant la boucle de calcul de l'algorithme du filtrage de Kalman :

### Phase de correction

Nous connaissons le vecteur d'état à l'instant *kₜ* prédit à l'instant *kₜ* - 1 *:* ***c̃***_{***kt**,****kt*-1**} et la matrice de covariance de l'erreur à l'instant *kₜ* prédite à l'instant *kₜ* - 1 : *P***_{*kt,kt*-1}** et nous observons ***yₖₜ.***

Nous voulons estimer : ***c̃_{kt,kt}***, ***P_{kt,kt}***

Le gain de Kalman ***Kₖₜ*** est déterminé avec la formule suivante où ***P*_{*kt,kt*-1}** est calculée dans l'équation 2D-44 ou 2D-45 : ${K}_{{k}_{t}} = {P}_{{k}_{t} , {k}_{t} - 1} {{H}^{˜}}^{T} {\left({H}^{˜}{P}_{{k}_{t} , {k}_{t} - 1}{{H}^{˜}}^{T}+R\right)}^{- 1}$

Le gain de Kalman exprime les poids (la confiance) donnés à la nouvelle mesure bruitée ***yₖₜ*** ou à l'estimée de l'état ***c***_{***kt**,****kt*-1**} qui est aussi soumise aux différentes perturbations externes. L'estimation de l'état actuel (estimateur d'état a posteriori) du système se fait en prenant une combinaison linéaire entre l'estimation réalisée à l'instant antérieur *kₜ* - 1 (estimateur d'état a priori) et la nouvelle donnée enregistrée. Le gain de Kalman intervient pour corriger l'estimation effectuée à l'instant *kₜ* - 1 pour l'instant actuel *kₜ* en fonction de la nouvelle mesure enregistrée ***yₖₜ**.*

Connaissant l'expression du gain de Kalman ***Kₖₜ*,** nous pouvons donner l'expression permettant d'estimer le vecteur d'état ***c̃****_{**kt**,**kt**}* à l'instant *kₜ* en fonction de la différence entre la mesure actuelle ***yₖₜ*** et la mesure estimée ***H̃c̃***_{***kt**,****kt*-1**} à partir de l'estimation précédente de l'état. Cette différence définit l'erreur de prédiction (***yₖₜ** -* ***H̃c̃***_{***kt**,****kt*-1**}) qui évalue la quantité de nouvelle information amenée par la mesure actuelle. ${{c}^{˜}}_{{k}_{t} , {k}_{t}} = {{c}^{˜}}_{{k}_{t} , {k}_{t} - 1} + {K}_{{k}_{t}} \left({y}_{{k}_{t}}-{H}^{˜}{{c}^{˜}}_{{k}_{t} , {k}_{t} - 1}\right)$

L'estimation de la matrice de covariance de l'erreur d'estimation à l'instant *kₜ* à partir de la matrice de covariance ***P***_{***kt**,****kt*-1**} estimée à l'instant *kₜ* - 1 se fait suivant la formule suivante : ${P}_{{k}_{t} , {k}_{t}} = \left(I-{K}_{{k}_{t}}{H}^{˜}\right) {P}_{{k}_{t} , {k}_{t} - 1} {\left(I-{K}_{{k}_{t}}{H}^{˜}\right)}^{T} + {K}_{{k}_{t}} R {K}_{{k}_{t}}^{T}$

L'estimation de ***c̃****_{**kt**,**kt**}* permet de connaître *C_{in blood},* ce dernier étant le terme de rang 1 du vecteur ***c̃****_{**kt**,**kt**}.*

A partir de *C_{in blood},* on peut estimer ${P}_{{CO}_{2}}^{in blood}$*.* ${P}_{{CO}_{2}}^{in sang} = \frac{{C}_{{CO}_{2}}^{in sang}}{{β}^{sang}}$

Où:
- *β^{sang}* est le coefficient de solubilité d'Ostwald du gaz carbonique dans le sang.

### Phase de prédiction

Nous connaissons le vecteur d'état ***c̃****_{**kt**,**kt**}* et la matrice de covariance de l'erreur d'estimation ***P_{kt,kt}*** à l'instant *kₜ*.

Nous souhaitons prédire le nouvel état du système ***c̃*_{*kt*+1,}*****ₖₜ**.* Nous nous plaçons dans le schéma d'intégration correspondant à la méthode implicite. L'équation de prédiction est la suivante : ${A}^{˜} {{c}^{˜}}_{{k}_{t} + 1 , {k}_{t}} = {{c}^{˜}}_{{k}_{t} , {k}_{t}} + {G}^{˜} {C}_{{k}_{t} + 1}^{in col air}$

Nous souhaitons aussi prédire la matrice de covariance de l'erreur ***P*_{*kt*+1}*****_{,kt}** :* ${P}_{{k}_{t} + 1 , {k}_{t}} = {{A}^{˜}}^{- 1} {P}_{{k}_{t} , {k}_{t}} {{A}^{˜}}^{- {1}^{T}} + {Q}^{˜}$

Grâce à ces formules, nous réalisons la prédiction à un pas à partir de l'équation d'état. Seulement un échantillon passé est retenu afin de prédire le comportement futur du système, à travers le vecteur d'état et la matrice de variance-covariance.

Bien que décrite en lien avec une mesure de CO₂ transcutanée, l'invention peut être mise en œuvre dans d'autres applications, de façon à mesurer un gaz émis par un milieu, solide ou liquide. Le milieu peut notamment être :
- un végétal, par exemple un fruit, de façon à suivre le procédé de maturation ;
- du lisier, de façon à suivre l'émission de gaz, par exemple le méthane ;
- un milieu de culture d'organismes ou de microorganismes biologiques ;
- de l'eau, par exemple de l'eau douce ou de l'eau de mer, par exemple pour suivre la concentration de gaz ou réguler la concentration de CO₂;
   un sol, de façon à en étudier la respiration.

## Revendications

1. Procédé d'estimation d'une teneur d'un gaz d'intérêt dans un milieu, à l'aide d'un dispositif de mesure, destiné à être disposé contre le milieu, le dispositif de mesure s'étendant entre une face de contact, destinée à être appliquée contre le milieu et une extrémité distale, le dispositif de mesure comportant une paroi latérale, s'étendant entre la face de contact et l'extrémité distale, le dispositif de mesure comportant :
- au niveau de la face de contact (10), au moins une ouverture d'admission (12), configurée pour collecter le gaz d'intérêt émis par le milieu, l'ouverture d'admission étant pratiquée à travers la face de contact;
- une chambre de mesure (20), comportant un capteur de gaz (23), le capteur de gaz étant configuré pour mesurer une concentration de gaz d'intérêt s'écoulant à travers la chambre de mesure;
- une chambre de collecte (30), reliée à la chambre de mesure, et délimitée par une ouverture sur la paroi latérale, la chambre de collecte comportant au moins une ouverture latérale (34), ménagée à travers la face latérale, ou sur une paroi supérieure de la chambre de collecte de façon à admettre de l'air ambiant dans la chambre de collecte ;
le dispositif étant tel que :
- la chambre de mesure (20) est disposée entre la face de contact (10) et la chambre de collecte (30) ;
- le dispositif comporte un moyen d'entraînement (41), configuré pour entraîner l'air, admis par l'ouverture latérale, à travers la chambre de collecte vers une ouverture d'évacuation (42), de façon que l'évacuation du gaz d'intérêt par l'air diminue la pression partielle de l'air dans la chambre de collecte, induisant un transport du gaz d'intérêt, par diffusion, de la face de contact vers la chambre de collecte, à travers la chambre de mesure :
- le procédé comportant :
- a) mesure de la concentration de gaz d'intérêt dans la chambre de mesure ;
- b) à l'aide d'une unité de traitement, modélisation du transport du gaz d'intérêt, entre le milieu et la chambre de collecte, la modélisation comportant une prise en compte de la diffusion du gaz depuis le milieu jusqu'à la chambre de collecte à travers la face de contact, à travers la chambre de mesure, ainsi qu'une convection du gaz dans la cellule de collecte résultant de l'entraînement produit par le moyen d'entraînement ;
- c) à partir de la mesure résultant de l'étape a), et d'une prise en compte du modèle résultant de l'étape b), estimation de la teneur du gaz d'intérêt dans le milieu ;
- et dans lequel
- le dispositif est spatialement discrétisé selon un maillage spatial, définissant des points de maillage entre la face de contact et la chambre de collecte ;
- le modèle est un modèle spatio-temporel discrétisé, de façon à estimer une teneur en gaz d'intérêt en différents points du maillage, et en différents instants .

2. Procédé selon la revendication 1, dans lequel l'étape b) comporte :
- modélisation de la diffusion du gaz d'intérêt à travers la chambre de mesure ;
- modélisation de la diffusion et de la convection du gaz d'intérêt dans la chambre de collecte.

3. Procédé selon l'une quelconque des revendications précédentes, dans lequel l'étape b) comporte une modélisation du transport du gaz d'intérêt dans le milieu.

4. Procédé selon l'une quelconque des revendications précédentes, dans lequel l'étape c) met en œuvre un estimateur récursif.

5. Procédé selon la revendication 4, dans lequel l'étape c) met en œuvre un estimateur récursif linéaire.

6. Procédé selon la revendication 5, dans lequel l'estimateur récursif linéaire est un filtre de Kalman.

7. Procédé selon l'une quelconque des revendications précédentes, dans lequel le milieu est la peau d'un utilisateur, la peau s'étendant en dessus d'un vaisseau sanguin, dans lequel l'étape b) comporte :
(i) à partir de la concentration de gaz d'intérêt dans la chambre de mesure, résultant de l'étape a), estimation d'une concentration de gaz d'intérêt transcutané ;
(ii) à partir de la concentration de gaz d'intérêt transcutané résultant de la sous-étape (i), estimation d'une concentration ou pression partielle de gaz d'intérêt dissous dans le sang de l'utilisateur.

8. Procédé selon l'une quelconque des revendications précédentes, comportant une modélisation du transport du gaz d'intérêt émis par le milieu à travers le dispositif.

9. Procédé selon l'une quelconque des revendications précédentes, dans lequel le milieu à analyser est un milieu solide ou liquide.

10. Procédé selon l'une quelconque des revendications précédentes, dans lequel la modélisation est basée sur un modèle bidimensionnel, le milieu, la chambre de mesure et la chambre de collecte étant discrétisés selon différents points de maillage répartis selon un axe parallèle à la face de contact et un axe perpendiculaire à la face de contact.

## Patentansprüche

1. Verfahren zum Schätzen eines Gehalts eines Gases von Interesse in einem Milieu mithilfe einer Messvorrichtung, die dazu bestimmt ist, an dem Milieu angeordnet zu werden, wobei sich die Messvorrichtung zwischen einer Kontaktfläche, die dazu bestimmt ist, an das Milieu angelegt zu werden, und einem distalen Ende erstreckt, wobei die Messvorrichtung eine Seitenwand umfasst, die sich zwischen der Kontaktfläche und dem distalen Ende erstreckt, wobei die Messvorrichtung umfasst:
- an der Kontaktfläche (10) mindestens eine Einlassöffnung (12), die dazu ausgestaltet ist, das von dem Milieu emittierte Gas von Interesse zu sammeln, wobei die Einlassöffnung durch die Kontaktfläche hindurch ausgebildet ist;
- eine Messkammer (20), die einen Gassensor (23) umfasst, wobei der Gassensor dazu ausgestaltet ist, eine Konzentration des durch die Messkammer hindurch strömenden Gases von Interesse zu messen;
- eine Sammelkammer (30), die mit der Messkammer verbunden ist und durch eine Öffnung in der Seitenwand begrenzt wird, wobei die Sammelkammer mindestens eine seitliche Öffnung (34) umfasst, die durch die Seitenfläche hindurch ausgebildet ist oder an einer oberen Wand der Sammelkammer, so dass sie Umgebungsluft in die Sammelkammer einlässt;
wobei die Vorrichtung dergestalt ist, dass:
- die Messkammer (20) zwischen der Kontaktfläche (10) und der Sammelkammer (30) angeordnet ist;
- die Vorrichtung eine Fördereinrichtung (41) umfasst, die dazu ausgestaltet ist, die durch die seitliche Öffnung eingelassene Luft durch die Sammelkammer hindurch zu einer Abführöffnung (42) zu fördern, so dass das Abführen des Gases von Interesse durch die Luft den Partialdruck der Luft in der Sammelkammer verringert, was zu einem Transport des Gases von Interesse durch Diffusion von der Kontaktfläche zu der Sammelkammer durch die Messkammer hindurch führt:
- wobei das Verfahren umfasst:
- a) Messen der Konzentration des Gases von Interesse in der Messkammer;
- b) mithilfe einer Verarbeitungseinheit, Modellieren des Transports des Gases von Interesse zwischen dem Milieu und der Sammelkammer, wobei das Modellieren eine Berücksichtigung der Diffusion des Gases von dem Milieu bis zu der Sammelkammer durch die Kontaktfläche hindurch, durch die Messkammer hindurch, umfasst sowie eine Konvektion des Gases in der Sammelzelle, die aus dem durch die Fördereinrichtung erzeugten Fördern resultiert;
- c) auf der Grundlage der aus Schritt a) resultierenden Messung und einer aus Schritt b) resultierenden Berücksichtigung des Modells, Schätzen des Gehalts des Gases von Interesse in dem Milieu;
- und wobei
- die Vorrichtung räumlich in ein räumliches Gitter diskretisiert ist, das Gitterpunkte zwischen der Kontaktfläche und der Sammelkammer definiert;
- das Modell ein diskretisiertes räumlich-zeitliches Modell ist, so dass ein Gehalt an einem Gas von Interesse an verschiedenen Punkten des Gitters und zu verschiedenen Zeitpunkten geschätzt wird.

2. Verfahren nach Anspruch 1, wobei der Schritt b) umfasst:
- Modellieren der Diffusion des Gases von Interesse durch die Messkammer hindurch;
- Modellieren der Diffusion und der Konvektion des Gases von Interesse in der Sammelkammer.

3. Verfahren nach einem der vorhergehenden Ansprüche, wobei der Schritt b) ein Modellieren des Transports des Gases von Interesse in dem Milieu umfasst.

4. Verfahren nach einem der vorhergehenden Ansprüche, wobei der Schritt c) einen rekursiven Schätzer implementiert.

5. Verfahren nach Anspruch 4, wobei der Schritt c) einen linearen rekursiven Schätzer implementiert.

6. Verfahren nach Anspruch 5, wobei der lineare rekursive Schätzer ein Kalman-Filter ist.

7. Verfahren nach einem der vorhergehenden Ansprüche, wobei das Milieu die Haut eines Verwenders ist, wobei sich die Haut über einem Blutgefäß erstreckt, wobei der Schritt b) umfasst:
(i) auf der Grundlage der Konzentration des Gases von Interesse in der Messkammer, die aus Schritt a) resultiert, Schätzen einer Konzentration eines transkutanen Gases von Interesse;
(ii) auf der Grundlage der Konzentration des transkutanen Gases von Interesse, die aus dem Teilschritt (i) resultiert, Schätzen einer Konzentration oder des Partialdrucks des Gases von Interesse, das in dem Blut des Verwenders gelöst ist.

8. Verfahren nach einem der vorhergehenden Ansprüche, umfassend ein Modellieren des Transports des Gases von Interesse, das von dem Milieu durch die Vorrichtung hindurch emittiert wird.

9. Verfahren nach einem der vorhergehenden Ansprüche, wobei das zu analysierende Milieu ein festes oder flüssiges Milieu ist.

10. Verfahren nach einem der vorhergehenden Ansprüche, wobei das Modellieren auf einem zweidimensionalen Modell basiert, wobei das Milieu, die Messkammer und die Sammelkammer an verschiedenen Gitterpunkten diskretisiert sind, die entlang einer parallel zu der Kontaktfläche verlaufenden Achse und einer senkrecht zu der Kontaktfläche verlaufenden Achse verteilt sind.

## Claims

1. Method for estimating a content of a gas of interest in a medium, using a measuring device, intended to be arranged against the medium, the measuring device extending between a contact face, intended to be applied against the medium and a distal end, the measuring device comprising a side wall, extending between the contact face and the distal end, the measuring device comprising :
- at the contact face (10), at least one inlet opening (12), configured to collect the gas of interest emitted by the medium, the inlet opening being made through the contact face;
- a measurement chamber (20), including a gas sensor (23), the gas sensor being configured to measure a concentration of gas of interest flowing through the measurement chamber;
- a collection chamber (30) connected to the measurement chamber and delimited by an opening in the side wall, the collection chamber having at least one side opening (34) through the side wall or in an upper wall of the collection chamber for admitting ambient air into the collection chamber;
the device being such that :
- the measurement chamber (20) is located between the contact face (10) and the collection chamber (30);
- the device includes drive means (41) configured to drive air, admitted through the side opening, and the gas of interest, by convection, through the collection chamber to a discharge opening (42), the air convection inducing transport of the gas of interest, by diffusion, from the contact face to the collection chamber, through the measurement chamber :
the method comprising
- a) measuring the concentration of the gas of interest in the measurement chamber ;
- b) by means of a processing unit, modeling of the transport of the gas of interest between the medium and the collection chamber, the modeling taking into account the diffusion of the gas of interest from the medium to the collection chamber through the contact face, through the measurement chamber, as well as convection of the gas of interest in the collection cell resulting from the entrainment produced by the entrainment means;
- c) from the measurement resulting from step a), and taking into account the model resulting from step b), estimation of the content of the gas of interest in the medium ;
and wherein
- the device is spatially discretized according to a spatial mesh, defining mesh points between the contact face and the collection chamber;
- the model is a discretized spatio-temporal model, so as to estimate a gas content of interest at different points in the mesh, and at different times;.

2. Method according to claim 1, wherein step b) comprises :
- modeling the diffusion of the gas of interest through the measurement chamber ;
- modeling the diffusion and convection of the gas of interest in the collection chamber.

3. Method according to any of the preceding claims, in which step b) involves modeling the transport of the gas of interest in the medium.

4. Method according to any of the preceding claims, in which step c) implements a recursive estimator.

5. Method according to claim 4, in which step c) implements a linear recursive estimator.

6. Method according to claim 5, in which the linear recursive estimator is a Kalman filter.

7. Method according to any one of the preceding claims, wherein the medium is the skin of a user, the skin extending over a blood vessel, wherein step b) comprises:
(i) from the gas concentration of interest in the measurement chamber resulting from step a), estimation of a transcutaneous gas concentration of interest;
(ii) from the transcutaneous gas concentration of interest resulting from sub-step (i), estimation of a concentration or partial pressure of dissolved gas of interest in the user's blood.

8. Method according to any of the preceding claims, including modeling the transport of the gas of interest emitted by the medium through the device.

9. Method according to any of the preceding claims, in which the medium to be analyzed is a solid or liquid medium.

10. Method according to any of the preceding claims, in which the modeling is based on a twodimensional model, the medium, the measurement chamber and the collection chamber being discretized according to different mesh points distributed along an axis parallel to the contact face and an axis perpendicular to the contact face.
